# EUROPEAN PATENT APPLICATION

(11) **EP 2 805 715 A1**
(43) Date of publication of application: **26.11.2014**
(21) Application number: 13305654.9
(22) Date of filing: 21.05.2013
(51) Int. Cl.: A61K 31/16, A61K 31/17, A61K 31/7088, A61P 31/18

(54) **Inhibitors of RNR2 subunit of ribonucleotide reductase to impair reverse transcription of hiv related lentiviruses in macrophages and use thereof for the treatment of hiv infected patients**

(71) Applicant: INSTITUT PASTEUR, 75724 Paris Cedex 15 (FR)
(72) Inventor: Pancino, Gianfranco, 75019 PARIS (FR); Allouch, Awatef, 94360 BRY SUR MARNE (FR); David, Annie, 92290 CHATENAY MALABRY (FR); Bergamaschi, Anna, 24050 Barlano (BG) (IT); Saez-Cirion, Asier, 92350 LE PLESSIS ROBINSON (FR); Barre-Sinoussi, Françoise, 92140 CLAMART (FR)
(74) Representative: Desaix, Anne

(57) **Abstract**

The invention relates to the use of inhibitors for the RNR2 subunit of the human ribonucleotide reductase to impair reverse transcription of HIV related lentiviruses in macrophages through down regulation of the intracellular dNTPs pool by repressing expression of RNR2. The invention also concerns a method of screening inhibitors of reverse transcription of HIV related lentiviruses in macrophages.

## Description

### Field of the invention

The invention relates to the use of inhibitors for the RNR2 subunit of the human ribonucleotide reductase to impair reverse transcription of HIV related lentiviruses in macrophages through down regulation of the intracellular dNTPs pool by repressing expression of RNR2. Macrophages targeted by the inhibitors used according to the invention are especially Monocyte Derived Macrophages (MDM). MDM encompass macrophages that are differentiated quiescent long-lived cells in the G0/G1 phase of cell cycle. Said inhibitors are used in the invention for the therapeutic treatment of human patients infected with a HIV lentivirus.

The invention also concerns a method of screening inhibitors of reverse transcription of HIV related lentiviruses in macrophages.

HIV related Lentiviruses of the invention encompass in particular human lentiviruses including HIV-1 and HIV-2 and simian lentiviruses including SIV.

### Background of the invention

HIV infection of macrophages greatly contributes to viral pathogenesis and progression to AIDS (recent review in (Verani, Gras et al. 2005). Macrophages are widely distributed in body tissues and can serve as vehicles for HIV-1 dissemination throughout the organs, including the lungs and the brain (Gartner, Markovits et al. 1986; Koenig, Gendelman et al. 1986). Because of their long half-life and relative resistance to the cytopathogenic effect of the HIV-1, macrophages may transmit the virus to other target cells, including CD4⁺ T lymphocytes, and contribute to the establishment of viral reservoirs (Orenstein, Fox et al. 1997; Aquaro, Bagnarelli et al. 2002; Sharova, Swingler et al. 2005). In addition, HIV-1 infection of macrophages affect their functions in innate and adaptive responses to pathogens, such as phagocytosis, intracellular pathogen killing and antigen presentation (reviewed in (Kedzierska, Azzam et al. 2003). However, the susceptibility of macrophages to HIV-1 infection varies greatly according to their differentiation status and tissue localization (Ancuta, Kunstman et al. 2006; Shen, Richter et al. 2009; Cassol, Cassetta et al. 2010). Environmental stimuli, such as cytokines, bacterial products as LPS, or circulating immune complexes bound to Fcγ receptors (FcγR) on macrophage membrane, may influence macrophage permissiveness to HIV-1 infection (Herbein, Coaquette et al. 2002). Moreover, HIV-1 replication in macrophages is regulated by cellular factors that can enhance or limit various steps of the viral life-cycle (Bergamaschi and Pancino 2010). In particular, HIV-1 reverse transcription in Monocyte Derived Macrophages (MDM) is much slower than in activated T cells, and this has been mainly attributed to the limited availability of nucleotide precursors in these non-dividing cells, in which the average of dNTP concentrations (∼0.026 µM) is about 200 times lower than in PHA-activated T cells (O'Brien, Namazi et al. 1994; Diamond, Roshal et al. 2004; Triques and Stevenson 2004). The recent identification of SAMHD1 as an HIV-1 restriction factor in dendritic cells and macrophages, counteracted by the HIV-2 and SIV Vpx protein that promotes its degradation (Hrecka, Hao et al. 2011; Laguette, Sobhian et al. 2011) supports this hypothesis. Indeed, SAMHD1 functions as a deoxynucleoside triphosphate (dNTP) triphosphohydrolase (Goldstone, Ennis-Adeniran et al. 2011; Powell, Holland et al. 2011), and the inventors have demonstrated that SAMHD1 degrades dNTPs keeping their concentration low and thus limiting HIV-1 replication in non-dividing cells (Lahouassa, Daddacha et al. 2012). Besides SAMHD1, other cellular factors have been reported to affect early post-entry steps of HIV-1 replication in macrophages, including the apolipoprotein B mRNA editing enzyme, catalytic polypeptide-like 3 (APOBEC3) cytidine deaminase family member APOBEC3A (Peng, Greenwell-Wild et al. 2007; Berger, Durand et al. 2011).

The inventors have previously shown that the p53-independent induction of p21^{Waf1/Cip1} (hereafter p21) by the aggregation of activating Fcγ receptors (FcγR) on MDM causes a strong decrease of reverse transcripts and integrated proviruses of HIV-1, HIV-2, SIVmac and SIVagm in MDM while siRNA-mediated p21 depletion rescued the viral replication (Bergamaschi, David et al. 2009). Remarkably, p21 limits HIV replication also in unstimulated MDM (Bergamaschi, David et al. 2009) and has been involved in the resistance of human hematopoietic stem cells to HIV-1 replication (Zhang, Scadden et al. 2007). In previous studies, the inventors have not detected p21 interactions with HIV-1 proteins associated to the reverse transcription and pre-integration complexes (RTC/PIC) that are implicated in the reverse transcription/integration steps (Bergamaschi, David et al. 2009) and Allouch A. unpublished results).

### Description of the invention

The inventors have now addressed the hypothesis that p21 may interfere with or modulate the expression of other cellular factor(s) necessary for these steps of HIV replication and have performed the present invention by identifying a cellular factor in macrophages that is regulated by p21 and that happens to interfere with reverse transcription of lentiviruses such as HIV or related viruses such as SIV.

p21 belongs to the Cip/Kip family of cyclin-dependent kinase inhibitors (CDKIs) (Gu Y 1993; Harper JW 1993; Xiong Y 1993). p21 was first described as a cell cycle inhibitor that blocks cell cycling at the G1/S interface and plays a critical role in the control of cell growth. p21 can influence cell cycle progression through pleiotropic mechanisms, including cyclin/CDK inhibition, binding to PCNA (Chen, Jackson et al. 1995), and control of the E2F transcription and activity (Sherr and Roberts 1995; Delavaine and La Thangue 1999). Although p21 is not a transcription factor, it may exert indirect effects on cellular gene expression. p21 induction in a fibrosarcoma cell line regulates the transcription of several genes, including genes involved in DNA replication, segregation and repair, either repressing or enhancing their expression (Chang, Watanabe et al. 2000).

MDMs are terminally differentiated, non-replicating and long-lived cells in which p21 is highly expressed comparing to cycling cells and plays important roles in monocyte differentiation and survival by inhibiting apoptosis (Liu, lavarone et al. 1996; Asada M 1999; Xaus J 1999; Steinman RA 2000; Coqueret O 2003).

The cellular factor accordingly identified by the inventors as impacting reverse transcription of lentiviral genome such as HIV or SIV RNA in macrophages is a subunit of a ribonucleotide reductase protein.

Ribonucleotide reductase (RR) is a multisubunit enzyme that catalyses the reduction of ribonucleotides to their corresponding deoxyribonucleotides and accordingly impacts the synthesis of DNA and its repair. Increased RR activity has been associated with malignant transformation of cells and tumor growth. Accordingly RR inhibitors have been targeted with a view to design anticancer chemotherapeutic agents and various molecules have been proposed including Triapine (WO 02/085358) Gemcitabine, GTI-2040. Properties of RR in relation with tumorigenesis and effect of their inhibitors in cancer treatment have been reported (Shao J. et al 2006). In human cells the expressed RR is an enzyme belonging to Class I enzymes and to subclass designated Class la, Class I enzymes being found in most eukaryotic cells. The human RR has two subunits designated R1 (large subunit) and R2 (small subunit) also designated RNR2 or RRM2 whose crystal structures have been determined separately in mouse cells for R1 and in E.coli for R2 (for R1: Uhling U. et al 1994; for R2: Nordlung P. et al 1993).In human cells, R2 has a homolog (80%) named p53R2 the expression of which follows two different pathways and impacts on dNTP supply in human cells. Also R2 and p53R2 show conservation of multiple functional domains but exhibit sequence differences including the fact that p53R2 lacks 33 amino acid residues in its N-terminal end with respect to R2 (Shao J. et al 2006).

The inventors have found that the R2 subunit of human ribonucleotide reductase (RNR2) may be targeted in order to inhibit lentiviral reverse transcription, illustrated with HIV and SIV lentiviruses, in quiescent cells and in particular in macrophages such as Monocyte Derived Macrophages (MDM). The obtained results were unexpected as it was previously found that RNR2 expression was thought to be restricted to S phase in dividing cells. From their experimental results the inventors were able to propose a novel and specific anti-lentiviral treatment strategy that would encompass targeting lentiviral replication to alter it in macrophages by inhibiting the reverse transcription of lentiviral genome and accordingly formation of lentiviral gene products in these cells.

Thus the invention provides means to inhibit lentiviral infection in macrophages and accordingly compounds and methods of use affecting viral reservoirs in patients infected with a lentivirus such as HIV-1 or HIV-2 and also impacting viral spread to other target cells.

The compounds and methods of use of the invention accordingly constitute means for treating HIV infection in a human patient, including for treating HIV reservoirs in various organs of tissues and/or for preventing dissemination of the HIV lentivirus in patient's organs or tissues.

The invention concerns a RNR2 inhibitor for use in the therapeutic treatment of a human patient infected with a HIV lentivirus, wherein the RNR2 inhibitor is administered in dosage conditions that enable it to interfere with reverse transcription of the lentiviral genome in HIV infected macrophages thereby altering lentiviral replication.

The sequence of the gene encoding human RNR2 is provided as SEQ ID No.13 and the amino acid sequence of the human RNR2 protein is provided as SEQ ID No.14.

By *"treatment"* or *"therapeutic treatment",* it is meant that the performed steps and dosage conditions of administration of a RNR2 inhibitor according to the invention result in improving the clinical condition of a human patient in need thereof, who has been diagnosed for infection by a HIV lentivirus and who suffers or may suffer in the future from disorder(s) associated with the infection as a result of chronic HIV infection. Such treatment aims at improving the clinical status of the human patient, by eliminating or alleviating the symptoms associated with the disorder(s) related to the HIV infection. In a preferred embodiment, the treatment according to the invention enables restoring to health.

"HIV lentivirus" encompasses human lentivirus of the HIV type including HIV-1 and HIV-2. "HIV related lentivirus" encompasses non-human lentiviruses such as simian lentivirus SIV, which has structural similarities with HIV-2 and in particular expresses Vpx protein.

The ability to "interfere "with reverse transcription of the lentivirus genome encompasses the capacity to restrict, to down regulate or to abolish or inhibit said reverse transcription in human macrophages.

Measurement of the effects of the administration of a RNR2 inhibitor according to the invention and thus of their capacity to interfere with reverse transcription may encompass measurement of the expression products of the HIV genes in particular measurement of the HIV-1 p24 protein or measurement of Late Reverse Transcription products of the HIV genome or may encompass measurement of the viral load. By *"viral load",* it is meant either the HIV RNA (which is derived from viral particles and present in plasma) or the HIV DNA (which is integrated in the cell genome and present in cells).

By *"HIV-infected patient"* it is meant a subject or patient who has been positively and accurately diagnosed for a HIV virus, and for whom HIV-infection has been confirmed following relevant testing. HIV-infected patients may be classified according to several parameters such as viral load, CD4 T cells number or clinical symptoms of AIDS.

In a preferred embodiment of the invention, a RNR2 inhibitor for use in the prevention or therapeutic treatment of a human patient infected with a HIV lentivirus according to the invention, targets macrophages that are differentiated non-replicating long-lived macrophages, in particular Monocyte Derived Macrophages (MDM).

Bone marrow-derived monocytes (Mos) are released into the blood where they circulate for a few days. Circulating Mos extravasate into the lungs, gastrointestinal tract, kidney, primary and secondary lymphoid organs and the central nervous system (CNS). In tissues, Mos undergo differentiation into tissue-specific macrophages (Mφ) and dendritic cells (DC).Resident tissue MΦ are long-lived cells with a half-life ranging from an average of 6 weeks (alveolar MΦ) to several years (resident brain MΦ).HIV-infected mononuclear phagocytes (bone marrow (BM) and blood Mo, tissue Mφ, microglia) can thus serve as vehicles for dissemination and reservoirs of HIV-1 infection (Crowe S et al 2003).

Monocyte Derived Macrophages (MDMs) are large phagocytic cells, which belong to the Reticuloendothelial System (RES) or Mononuclear Phagocyte System (MPS) and are derived from blood monocytes. MDMs can also be divided as fixed or free macrophages: fixed macrophages such as histiocytes of connective tissue or Kupffer cells of liver and spleen, cells of lymph nodes... and free macrophages in inflammatory regions.

A particular subgroup of Monocyte Derived Macrophages (MDMs) of interest in accordance with the invention is MDMs that express the following markers: CD14, Fcγ receptors, differentiation markers including CD11b and/or CD71 and M2 macrophage polarization markers including CD163 and/or CD206.

The invention also relates to a RNR2 inhibitor for use in the prevention of constitution of HIV reservoirs or in the therapeutic treatment of replenishment of HIV reservoirs, in differentiated non-replicating macrophages in a human patient infected with a HIV lentivirus, wherein the RNR2 inhibitor is administered in dosage conditions that enable it to impair reverse transcription of the lentiviral genome in Monocyte Derived Macrophages of the patient.

The invention also relates to a RNR2 inhibitor for use in the prevention or the therapeutic treatment of dissemination of the HIV lentivirus in a human patient infected with said HIV lentivirus, in particular for the prevention or the treatment of lentivirus dissemination in peripheral organs such as lungs, brain or liver or the prevention or treatment of lesions in peripheral organs such as lungs, brain or liver, wherein the RNR2 inhibitor is administered in dosage conditions that impair reverse transcription of the lentiviral genome in Monocyte Derived Macrophages of the patient.

In a particular embodiment of the invention, a RNR2 inhibitor for use in the treatment of a human patient infected with a HIV lentivirus according to the invention is administered in dosage conditions, which do not affect Monocyte Derived Macrophages viability.

Examples of RNR2 inhibitors for use in the treatment of a human patient infected with a HIV lentivirus according to the invention, are selected among radical scavengers, iron chelators, antisense oligonucleotides, and interfering RNA.

In particular, RNR2 inhibitor for use in the treatment of a human patient infected with a HIV lentivirus according to the invention, are selected among Hydroxyurea (specific inhibitor of DNA synthesis *in vivo,* CH₄N₂O₂)(Merck Index 12th edition Entry 4898), and Deferoxamine mesylate (aluminum and iron (III) chelator, C₂₅H₄₈N₆O₈.CH₄O₃S)(Merck Index 12th edition Entry 2914).

RNR2 inhibitors such as Hydroxyurea or Deferoxamine mesylate may be administered in dosage conditions that lead to a concentration in the targeted cells, which are lower than those usually showing hematological toxicity when used for antitumor activity.

Antisense oligonucleotides having an activity as RNR2 inhibitor encompass GTI-2040. GTI-2040 is a 20-mer oligonucleotide that is complementary to a coding region in the mRNA of the RNR2 subunit of human ribonucleotide reductase (Lee Y et al 2003).

Small interfering RNAs (siRNA) are disclosed in the examples which follow and can be used as a pool of siRNA. In particular according to the invention, siRNA are those having the sequence disclosed as SEQ ID No. 1 to 4. The examples provided herein show that these siRNA, optionally used as a pool, prevent the transcription of the RNR2 gene in macrophages such as MDMs.

According to a preferred embodiment of the invention, RNR2 inhibitor for use in the treatment of a human patient infected with a HIV lentivirus according to the invention, is used in a human patient who has been diagnosed for early phase infection with HIV, especially before Setpoint of HIV replication.

The invention also relates to a RNR2 inhibitor for use in the treatment of a human patient infected with a HIV lentivirus, where the patient is under combination antiretroviral therapy.

In particular, a RNR2 inhibitor for use in the treatment of a human patient infected with a HIV lentivirus is used in combination antiretroviral therapy (cART) with a acyclic nucleoside such as acyclovir or gancyclovir, nucleoside analog, such as dideoxyinoside (DDI) and/or with 3'-azido-3'-deoxythymidine (AZT), and/or a reverse transcriptase inhibitor and/or a protease inhibitors and/or integrase inhibitors.

In a particular embodiment a RNR2 inhibitor for use in the treatment of a human patient infected with a HIV lentivirus according to the invention, is used in a patient who has been diagnosed for neurological disorders.

The expression *"neurological disorders" associated with HIV infection"* encompasses neurological disorders of the nervous system which are caused directly by the HIV virus, by certain cancers and/or opportunistic infections, as well as disorders of unknown origin which are influenced by but are not known to be caused directly by the virus. Some of these neurological disorders associated with AIDS may be characteristic of the state of progression of the disease. Examples of neurological disorders associated with AIDS are AIDS dementia complex (ADC) or HIV-associated encephalopathy, central nervous system lymphomas, cryptococcal meningitis, cytomegalovirus (CMV) encephalitis, encephalitis and myelitis caused by the herpes zoster virus, neuropathy (peripheral neuropathy and distal sensory polyneuropathy), neurosyphilis, progressive multifocal leukoencephalopathy (PML), toxoplasma encephalitis or cerebral toxoplasmosis and vacuolar myelopathy. Some patients with controlled viral load (VL) who are not at the AIDS stage, as for example patients under potent antiretroviral therapy (HAART) or cART may develop neurological disorders.

The invention also concerns a pharmaceutical formulation comprising an inhibitor of the RNR2 subunit of the ribonucleotide reductase in an amount sufficient to inhibit HIV replication in differentiated, non-replicating macrophages in a human patient infected with a HIV lentivirus.

A particular pharmaceutical formulation for use according to the invention in therapeutically treating a human patient previously diagnosed for infection by a HIV lentivirus, is a formulation which comprises active ingredients including a RNR2 inhibitor in combination with a pharmaceutical carrier or diluents, wherein the RNR2 inhibitor is present in the formulation in a form and amount adapted to release RNR2 inhibitor after administration to the patient, such that RNR2 inhibitor release causes RNR2 inhibitor to be detectable in the plasma of the patient in a concentration that does not induce hematological toxicity, in particular in a concentration of 0.1 to 2µM, in particular 0.2 to 1µM of the RNR2 inhibitor in plasma.

A pharmaceutical composition according to the invention of for use according to the invention is one where the RNR2 inhibitor is selected among radical scavengers, iron chelators antisense oligonucleotides and interfering RNA, in particular the RNR2 inhibitor is Hydroxyurea or Deferoxamine mesylate. The particular examples provided in the present application are examples of active ingredient for such pharmaceutical formulations.

The RNR2 inhibitor may be administered by any suitable administration route including parenteral and in particular intravenous route or intramuscular or via mucosal administration or via oral route.

The invention thus also relates to a method of therapeutically treating a human patient previously diagnosed for infection by a HIV lentivirus, comprising:
- administering a RNR2 inhibitor in dosage conditions that enable it to interfere with reverse transcription of the lentiviral genome in HIV infected macrophages thereby altering lentiviral replication *in vivo,*
- following the clinical condition of the treated patient.

The embodiments disclosed herein in relation to the use of the RNR2 inhibitors according to the invention apply to the method of treating defined above. In particular, the method of therapeutically treating a human patient previously diagnosed for infection by a HIV lentivirus may be part of a cART.

The invention also relates to a method of *in vitro* screening RNR2 inhibitors candidates for their capacity to interfere with reverse transcription of a HIV or SIV lentiviral genome in human differentiated non-replicating macrophages, in particular in Monocyte Derived Macrophages, comprising the steps of:
- providing in a macrophages culture medium, differentiated Monocyte Derived Macrophages (MDMs) that express the following markers: CD14, Fcγ receptors, differentiation markers including CD11b and/or CD71 and M2 macrophage polarization markers including CD163 and/or CD206;
- infecting MDMs with a HIV retrovirus or a derivative thereof wherein said HIV or derivative optionally expresses a reporter gene;
- contacting the infected MDMs with determined concentrations of a candidate RNR2 inhibitor;
- determining HIV replication gene products, in particular measuring the expression or the activity of the reporter gene, thereby determining efficacy of RNR2 inhibitor candidate to block HIV infection of MDMs.

A macrophage culture medium may be RPMI 1640 supplemented with 200mM L-glutamine, 100 U of penicillin, 100 µg of streptomycin, 10mM HEPES, 10mM sodium pyruvate, 50 µM β-mercaptoethanol, 1% minimum essential medium vitamins, 1% non essential amino acids.

A method of *in vitro* screening RNR2 inhibitors for their capacity to interfere with reverse transcription of a HIV or SIV lentiviral genome in human differentiated non-replicating macrophages, in particular in Monocyte Derived Macrophages, according to the invention may further comprise steps for preparation of MDMs including:
- providing buffy coat of anticoagulated blood sample of a healthy donor;
- differentiating monocytes of the buffy coat into macrophages and harvesting Monocyte Derived Macrophages (MDMs) that express the following markers: CD14, Fcγ receptors, differentiation markers including CD11b and/or CD71 and M2 macrophage polarization markers including CD163 and/or CD206;
- resuspending MDMs in macrophage culture medium.

The HIV virus used for the infection of the macrophages is a replicating virus. Its replication may be detected and possibly quantified by measurement of HIV gene products (such as p24) or LRTs (Late Reverse Transcripts). Quantification of LRTs may be performed by quantitative PCR (RT-qPCR).

According to a particular embodiment of the invention, the infecting HIV virus used to perform the screening method is a pseudotyped HIV virus such as a VSV-G pseudotyped virus, expressing a reporter gene such as the luciferase gene.

Antiviral activity after infection with a VSV-G pseudotyped HIV virus that expresses luciferase can be easily assessed by measure of luciferase activity with a luminometer or by bioluminescence.

The technique for quantification of bioluminescence in infected cells has been described in the examples. Briefly, the system includes a fully automated inverted microscope (200M, Carl Zeiss, Germany) and is housed in a light-tight dark box. Low level light emission was collected using an Imaging Photon Detector (IPD 3, Photek Ltd. East Sussex, UK) and brightfield images were obtained with a CCD camera (Coolsnap HQ, Roper Scientific). Observations were made with Plan-Neofluar 10X (air, NA=0.5), 25X (oil, NA=0.8) objectives (Carl Zeiss, Germany). The data acquisition software allowed superposition of photon events with brightfield images and selective quantification of photons in delimited surfaces. 10⁵ MDM were plated after infection on glass-bottomed dishes (MatTek corp, MA, USA), washed and cultured in complete medium for 5 days before analysis. The analyses were conducted in PBS + 5% FCS or complete phenol red free medium at room temperature in presence of endotoxin free beetle D-luciferin (Promega) (final concentration 1 mM).

In a particular embodiment, a method of *in vitro* screening according to the invention, further comprises a step of assaying specificity of the candidate RNR2 inhibitor for targeting RNR2.

The invention will be further disclosed in the figures and examples which follow, especially for the description of particular embodiments or features that are transposable to the above defined compounds and methods.

### FIGURE LEGENDS

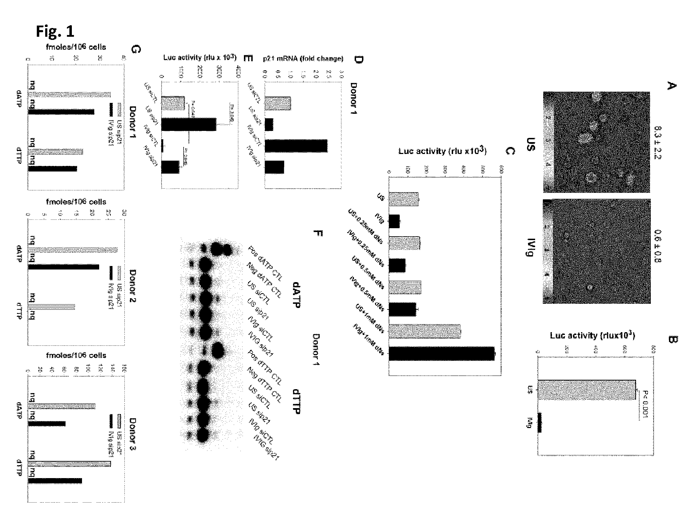

**Figure 1****. Exogenous deoxynucleosides (dNs) rescue p21-mediated restriction of HIV-1 and p21 knockdown increases dNTP levels in MDM.** (A-B) Unstimulated (US) and IVIg-activated MDM were infected with HIV-1/VSV-G 2 h after IVIg stimulation. HIV-1 replication was quantified at five days post infection in living cells by bioluminescence imaging of the infected living MDM (integration = 80 seconds) (A).The numbers of positive cells indicated above the images are means ± s.d. of 9 and 13 random fields for US and IVIg MDM respectively. In parallel samples, HIV-1 replication was quantified by measuring luciferase activity in lysates of infected MDM (B). (C) Unstimulated (US) and IVIg-activated MDM were infected with HIV-1/VSV-G 2 h after IVIg stimulation and were then treated with increasing amounts of dNs (0.25-1 mM) for 24 hours. HIV-1 replication was determined by measuring luciferase activity 72 hours post infection (hpi). The data are means ± standard deviation of triplicate wells. (D-F) Unstimulated (US) and IVIg activated MDM from one donor were p21-silenced by transfection with p21 siRNAs (sip21), while control MDM were transfected with a pool of non-targeting siRNAs (siCTL). At 22 h post-siRNAs transfection and 24 hours post IVIg stimulation: p21 mRNA was quantified by qRT-PCR (D); MDM were infected with HIV-1/VSV-G and viral replication was determined by luciferase activity at 72 hpi (E). The data are means ± standard deviation of triplicate wells; MDM were lysed for the quantification of intracellular dATP and dTTP using the single-nucleotide incorporation gel analysis (Diamond T. et al 2004) (F). (G) The graphs represent the mean of duplicate absolute values in fmoles/million cells of intracellular dATP and dTTP in p21 knockdown (sip21) unstimulated (US) and IVIg-activated MDM from three different donors. dNTP levels in unstimulated and IVIg-activated MDM treated with control siRNA (siCTL) were too low to allow quantification and are indicated as non-quantifiable (nq).

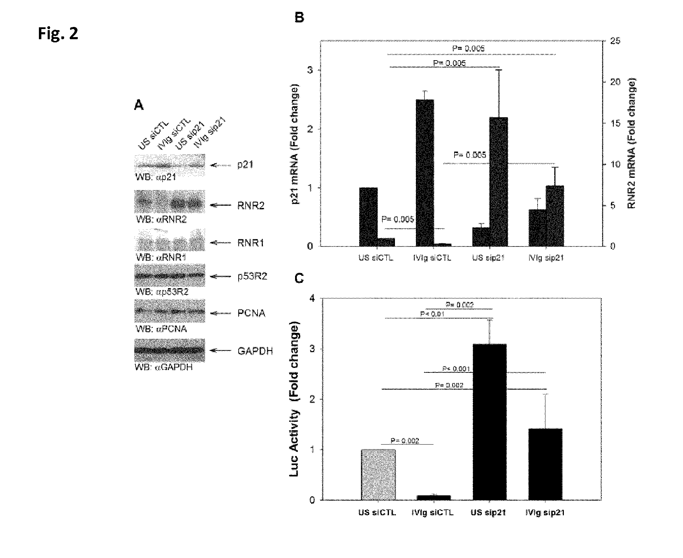

**Figure 2****. p21 inhibits the expression of the metabolic dNTP enzyme RNR2.** (A) Unstimulated (US) and IVIg activated MDM were transfected with p21 siRNA (sip21) and a pool of non-targeting control siRNAs (siCTL). At 24 h after IVIg stimulation and 22 h after siRNA transfection, MDM were analyzed by Western blot (WB) for p21, RNR2, RNR1, p53R2, PCNA and GAPDH protein expression, using the indicated antibodies. (B) p21 and RNR2 mRNA from unstimulated and IVIg-activated control (siCTL) and p21 silenced (sip21) MDM were quantified by qRT-PCR. The data are the fold change (column left: p21 mRNA; column right: RNR2 mRNA) with respect to control cells (unstimulated siCTL) ± standard error of MDM from five donors. (C) In parallel, unstimulated and IVIg activated siCTL and sip21 MDM were infected with HIV-1/VSV-G. HIV-1 replication was determined by measuring luciferase activity 72 hpi. The data are the fold change with respect to control MDM (unstimulated siCTL) ± standard error for MDM from six donors.

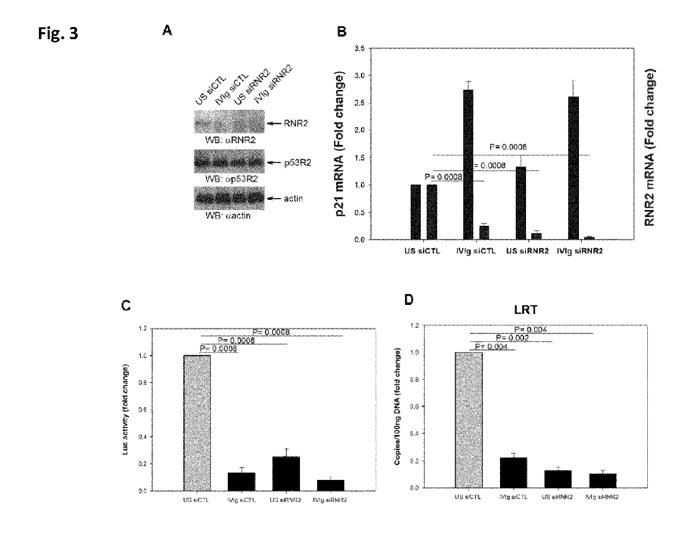

**Figure 3****. RNR2 knockdown inhibits HIV-1 reverse transcription.** (A) Unstimulated (US) and IVIg activated MDM were transfected with a pool of four siRNAs against the RNR2 gene (siRNR2) or with a pool of non-targeting control siRNAs (siCTL). At 24 h after IVIg stimulation and 22 h after siRNA transfection, MDM were analyzed by WB with antibodies against RNR2, p53R2 and actin. (B) RNR2 (column right) and p21 (column left) mRNAs in unstimulated (US) and IVIg activated siCTL and siRNR2 MDM were quantified by qRT-PCR. The data are the fold change with respect to control MDM (siCTL unstimulated) ± standard error for MDM from seven donors. (C) Unstimulated and IVIg activated siCTL and siRNR2 MDM were infected with HIV-1/VSV-G. Viral replication was determined by measuring luciferase activity 72 hpi. The data are the fold change with respect to control MDM (siCTL unstimulated) ± standard error for MDM from seven donors. (D) Unstimulated and IVIg activated siCTL and siRNR2 MDM were infected with HIV-1/VSV-G. Reverse transcription was measured by quantifying late reverse transcripts (LRTs) at 72 hpi by means of qRT-PCR. The data are the fold change with respect to control MDM (siCTL unstimulated) ± standard error for MDM from six donors.

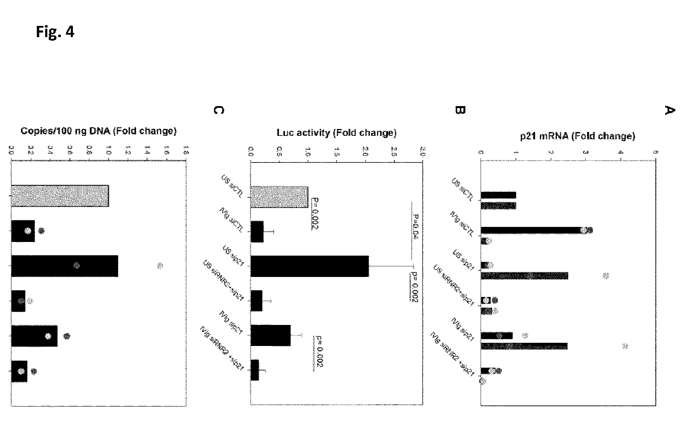

**Figure 4****. p21 inhibits HIV-1 reverse transcription by down-regulating RNR2.** (A) Unstimulated and IVIg-treated MDM from two donors were transfected with siRNA against the p21 gene (sip21) or co-transfected simultaneously with sip21 and a pool of four siRNAs against the RNR2 gene (sip21+siRNR2). Control MDM were transfected with a pool of non-targeting siRNAs (siCTL). At 24 h after IVIg stimulation and 22 h after siRNA transfection, p21 and RNR2 mRNAs were quantified by qRT-PCR. The data are the mean fold change with respect to control MDM (unstimulated siCTL) in the two donors. Light grey circles and grey circles represent the values for each donor. (B) Unstimulated and IVIg-treated MDM (siCTL, sip21 and sip21+siRNR2) at 24 hours post IVIg activation and 22 hours post siRNA transfection were infected with HIV-1/VSV-G. Viral replication was determined by measuring luciferase activity 72 hpi. The data are the mean fold change with respect to control MDM (siCTL unstimulated) in the two donors. (C) HIV-1 reverse transcription in unstimulated and IVIg-treated siCTL, sip21, and sip21+siRNR2 MDM infected with HIV-1/VSV-G was determined by quantifying LRTs at 72 hpi by qRT-PCR. The data are the mean fold change with respect to control MDM (unstimulated siCTL) in the two donors. Light grey circles and grey circles represent the values for each donor.

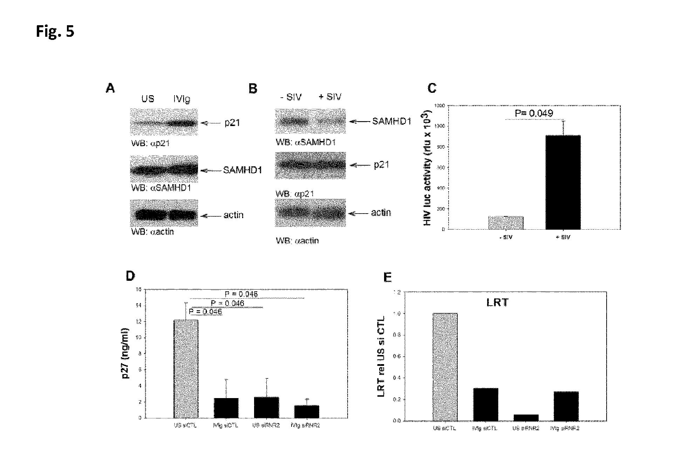

**Figure 5****. p21-mediated restriction is unrelated to SAMHD1 and inhibits SIVmac251 through RNR2 suppression.** (A) Unstimulated (US) and IVIg activated MDM were controlled by WB for p21, SAMHD1 and actin expressions at 24 hours post IVIg activation using the indicated antibodies. (B-C) MDM were infected with HIV-1/VSV-G or co-infected with HIV-1/VSV-G and SIVmac251 (+SIV). MDM were controlled for SAMHD1, p21 and actin expressions at 24 hpi (B) and for HIV-1 replication by measuring luciferase activity at 72 hpi (C). The data are means ± standard deviation of triplicate wells. (D) Unstimulated and IVIg activated MDM from one donor were silenced for RNR2 by transfecting four specific siRNAs (siRNR2) and infected with SIVmac251 at 22 h post siRNAs transfection and 24 h post IVIg activation. Control MDM were transfected with non-targeting control siRNA (siCTL). Viral replication was measured by quantifying p27 antigen 7 days post-infection. The data are means ± standard deviation of triplicate wells. These data are representative of experiments with MDM from 3 donors. (E) SIV LRTs were determined by qRT-PCR at 48 hpi.

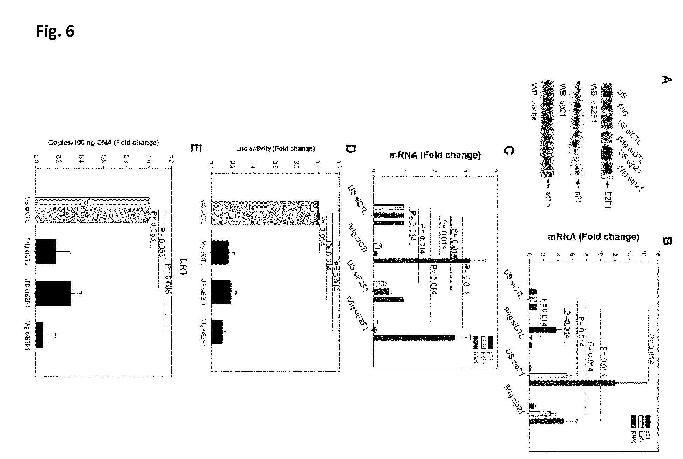

**Figure 6****. p21 represses RNR2 transcription by down-regulating E2F1.** (A) Unstimulated (US) and IVIg activated MDM were transfected with p21 siRNA (sip21) and a pool of non-targeting control siRNAs (siCTL). At 24 h after IVIg stimulation and 22 h after siRNA transfection, MDM were analyzed by WB for E2F1, p21 and actin protein expression. (B) p21 (column left), E2F1 and RNR2 (column right) mRNAs from siCTL and sip21 (unstimulated or IVIg-treated) MDM were quantified by qRT-PCR 24 h after IVIg stimulation and 22 h after siRNA transfection. The data are the fold change with respect to control cells (unstimulated siCTL) ± standard error for MDM from four donors. (C) unstimulated and IVIg activated MDM were transfected with a pool of four siRNAs against the E2F1 gene (siE2F1) or with control siRNAs (siCTL). At 24 h after IVIg stimulation and 22 h after siRNA transfection, E2F1, RNR2 (column right) and p21 (column left) mRNAs were quantified by qRT-PCR. The data are the fold change with respect to control MDM (siCTL unstimulated) ± standard error for MDM from four donors. (D) siCTL and siE2F1 (unstimulated or IVIg-treated) MDM were infected with HIV-1/VSV-G. Viral replication was quantified by measuring luciferase activity at 72 hpi. The data are the fold change with respect to control MDM (siCTL unstimulated) ± standard error for MDM from four donors. (E) HIV-1 reverse transcription in siCTL and siE2F1 (unstimulated or IVIg) MDM infected with HIV-1/VSV-G was measured by quantifying LRTs by qRT-PCR 72 hpi. The data are the fold change with respect to control MDM (siCTL unstimulated) ± standard error for MDM from three donors.

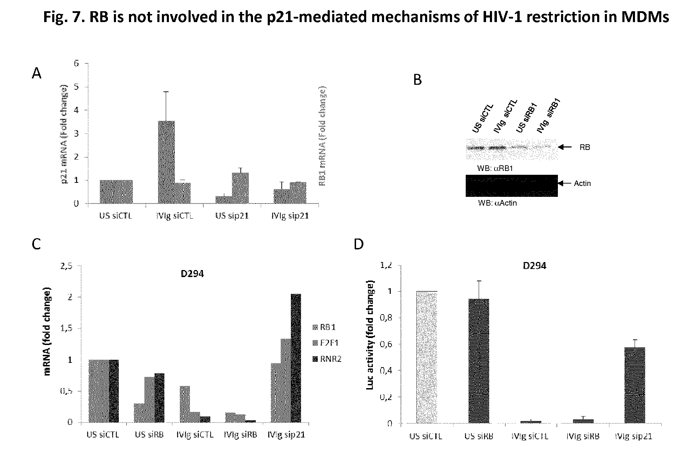

**Figure 7****. RB (retinoblastoma protein) is not involved in the p21-mediated mechanisms of HIV-1 restriction in MDMs. (A)** Un-stimulated (US) and IVIg MDMs were transfected with siRNA against p21 gene (sip21) or with a non-targeting pool control siRNAs (siCTL). At 24 hours post IVIg stimulation and 22 hours post siRNAs transfection p21 (column left) and RB1 (column right) mRNAs were quantified by qRT-PCR. The data are represented in fold change with respect to control MDMs (siCTL US) ± standard errors of experiments on three different donors. (B) US and IVIg stimulated MDMs were transfected with either a pool of four siRNAs against RB gene (siRB) or with a pool of non-targeting siRNAs (siCTL). At 48 hours post IVIg stimulation and 46 hours post siRNAs transfection, MDMs were analyzed by Western Blot (WB) with anti-RB and anti-Actin antibodies. (C) US and IVIg MDMs were transfected with siRB and siCTL siRNAs. The IVIg MDMs were also transfected with the sip21 siRNA. At 24 hours post IVIg stimulation and 22 hours post siRNAs transfection RB (left grey column), E2F1 (light grey column) and RNR2 (black column) mRNA were quantified by qRT-PCR. (C) siCTL (US and IVIg), siRB (US and IVIg) and sip21 (IVIg) MDMs were infected with HIV-1/VSV-G at 24 hours post IVIg stimulation and 22 hours post-siRNAs transfection. The HIV-1 replication was determined by the Luciferase activity at 72 hpi. The data are represented in fold change with respect to control MDMs (siCTL US) ± standard deviations of triplicate wells.

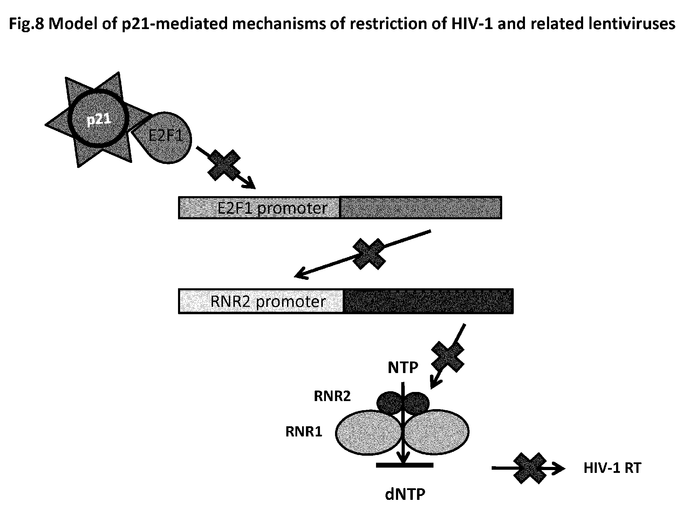

**Figure 8****. Model of p21-mediated restriction of HIV-1 and related lentiviruses in MDMs.** p21 inhibits E2F1 expression. The RNR2 transactivation is consequently inhibited. The RNR2 down-regulation decreases the dNTPs biosynthesis. The HIV-1 cDNA synthesis is blocked due the unavailability of dNTPs for reverse transcription (RT) catalysis.

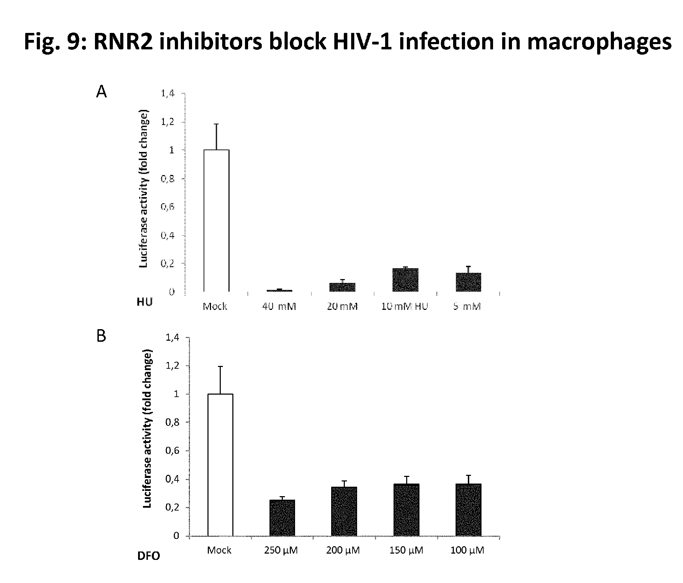

**Figure 9****. RNR2 inhibitors block HIV-1 infection in macrophages.** Dose dependent inhibition of HIV-1 replication by RNR2 inhibitors: Monocyte-derived-macrophages were infected with HIV-1/VSV-G and then treated with decreasing concentrations of hydroxyurea (HU) (40 to 4 mM) (A) or Deferoxamine Mesylate (DFO) (250 to 100 µM) (B). HIV-1 replication was determined by measuring luciferase activity 72 hours post infection. Results are expressed as fold changes of luciferase activity in drug-treated cells with respect to mock treated cells. The data are means ± standard deviation of triplicate wells.

### EXAMPLES

### I. p21-mediated RNR2 Repression Restricts HIV-1 Replication in Macrophages by inhibiting dNTP biosynthesis pathway

The inventors investigated whether p21 could lower the dNTP pool in macrophages below the levels needed for HIV-1 reverse transcriptase processivity interfering with dNTP metabolic pathways.

### RESULTS

### The p21-mediated blocking of HIV-1 reverse transcription is relieved by the supply of exogenous deoxynucleosides to MDMs

The phenotype of HIV-1 restriction associated to p21 expression in MDM that we have previously described (Bergamaschi, David et al. 2009)is resumed by the experiment shown in immunoglobulins (IVIg) inhibits MDM transduction by a VSV-G pseudotyped NL4.3-Luc HIV-1 virus (HIV-1/VSV-G), containing a reporter *luciferase* gene, while p21 knockdown (sip21) rescues viral replication both in un-stimulated (US) or IVIg stimulated MDMs. We have previously shown that the major blocking effect caused by p21 in HIV-1 replication is at the level of reverse transcription (Bergamaschi, David et al. 2009). It is known that the delivery of extracellular deoxynucleosides (dNs) as dNTP precursors accelerates HIV-1 reverse transcription in MDMs by increasing the intracellular dNTPs pool that is a limiting factor in these cells (Collin and Gordon 1994; Furge and Guengerich 1997; Diamond, Roshal et al. 2004). Exogenous dNs are taken up by the cells and converted into dNTPs through the salvage pathway of the dNTPs synthesis, which results in a larger intracellular dNTP pools (Meyerhans, Vartanian et al. 1994; O'Brien, Namazi et al. 1994; Jamburuthugoda, Chugh et al. 2006). We therefore investigated whether the addition of dNs to MDM cultures was able to counteract the reverse transcription inhibition induced by p21. To this aim, MDMs from nine donors were activated by seeding on immobilized IVIg or left unstimulated (US) and were infected with HIV-1/VSV-G. Pyrimidine and purine dNs were subsequently added or not to the culture medium. HIV-1 replication was quantified by measuring luciferase activity at 72 hours post-infection (Hpi) (Figure 1A). As expected, IVIg stimulation of MDMs significantly inhibited HIV-1 replication (Figure 1B). In contrast, dNs strongly enhanced HIV-1 replication in US MDMs (mean of 9: 3.2 folds), consistently with our previous report (Lahouassa, Daddacha et al. 2012). Remarkably, dNs treatment was also able to rescue HIV-1 replication in the IVIg activated MDMs to levels comparable to those of the dNs treated US MDMs (Figure 1B). The treatment of the IVIg-activated MDMs with increasing amounts of dNs (0.25-1.5 mM) relieved the viral blocking of HIV-1 replication in a dose dependent manner(Figure 1C). Thus, dNs supply is able to relieve the p21-mediated HIV-1 restriction in MDMs. This suggests that p21 inhibits HIV-1 replication by affecting negatively the dNTP pools available for HIV-1 reverse transcription.

### p21 down-regulates the dNTPs concentrations in MDMs

We then investigated whether p21 expression modulates the intracellular dNTP pool. Intracellular dNTP concentrations were quantified in unstimulated and in IVIg stimulated MDM in which p21 had been silenced by transfection with a small interfering RNA (siRNA) targeting the p21 gene (sip21). Control cells were transfected with a non-targeting siRNA pool (siCTL). Figures 1D-F show results for cells from a representative donor (donor 1). The efficiency of p21 silencing, determined by p21 mRNA quantitative real-time PCR (qRT-PCR), was 72% and 83% in sip21 unstimulated MDM and in IVIg stimulated MDM, respectively (Figure 1D). As expected, HIV-1/VSV-G infection of p21-silenced MDM (sip21) was more efficient than in control cells (siCTL) (Figure 1E). Intracellular dNTP concentrations were measured with a single-nucleotide incorporation assay (11, 19). dNTP levels in unstimulated and IVIg stimulated MDM treated with control siRNA (siCTL) were too low to allow quantification (Figure 1F) and thus are indicated as non-quantifiable (nq) in Figure 1G. Remarkably, dATP and dTTP intracellular concentrations increased to quantifiable levels after p21 knockdown (sip21) (Figure 1F and Figure 1G). Results from three different donors showed that, in sip21-knockdown unstimulated MDM, dATP and dTTP concentrations, expressed as femtomoles per million cells, were 34.3, 27.8, 111.3 and 22.8, 14.6, 136.8, for donor 1, 2 and 3 respectively (Figure 1G). In sip21-knockdown IVIg stimulated MDM, dATP concentrations were 27.6, 22.1, 62 for donor 1, 2 and 3 respectively, and dTTP concentrations, quantifiable in two donors, were 20.4 and 90 for donor 1 and 3 (Figure1G). These results show for the first time that p21 interferes with dNTP metabolism in MDM, depleting the dNTP pool and thereby restricting HIV-1 replication.

### p21 inhibits the expression of the ribonucleotide reductase subunit R2: RNR2

We then asked whether the p21-mediated down-regulation of dNTP pool size was due to an interference of p21 with the dNTP biosynthesis pathways in MDMs. To this aim the expressions of the proteins involved in the *de novo* synthesis of dNTPs (RNR1, RNR2 and p53R2) were analyzed by Western Blot (WB) in US MDMs silenced or not for p21 and as a control in the IVIg-stimulated MDMs. The expressions of the p21 interacting protein PCNA and of the housekeeping protein GAPDH were also analyzed as a further control and for normalization of the samples respectively. As shown in Figure 2A IVIg stimulation increased, as expected, p21 protein levels whereas p21 siRNAs (sip21) mediated knockdown reduced p21 in comparison to US MDMs transfected with control siRNA (siCTL). RNR1 and p53R2 protein expressions remained unchanged whatever p21 levels increased or decreased (Figure 2A). In contrast, RNR2 protein was depleted upon p21 induction and was strongly increased after p21 knockdown (sip21) (Figure 2A). Following p21 silencing no variation in MDM number was observed in comparison with siCTL transfected cells. The expression levels of PCNA and GAPDH were unchanged indicating equal amounts of proteins were loaded (Figure 2A). These data are representative of experiments with MDM from three different donors (data not shown) and demonstrate that p21 down-regulates the expression levels of RNR2 protein in MDMs. To investigate whether p21 regulates RNR2 expression at transcriptional level RNR2 and p21 mRNAs were quantified by qRT-PCR under the same conditions described above. Results with MDM from five different donors showed that, similarly to the proteins, RNR2 mRNA levels were significantly (p=0.005) decreased (60 to 90%) in parallel with the increase of p21 mRNA following IVIg stimulation of MDMs. In contrast, RNR2 mRNA was significantly (p=0.005) increased (5 to 30 fold) in MDMs depleted of p21 (sip21) (Figure 2B). The opposite variations of p21 and RNR2 expressions were well correlated with the effects of p21 induction or depletion on HIV-1 replication in all the donors (Figure 2C). After MDMs infection with HIV-1/VSV-G, in the IVIg stimulated MDMs (high p21 and low RNR2) HIV-1 replication was inhibited while in the p21 knockdown MDMs (low p21 and high RNR2) HIV-1 replication was enhanced (Figure 2C). These results suggest that p21 inhibits the reverse transcription of HIV-1 by blocking dNTPs biosynthesis through the inhibition of RNR2 expression.

### RNR2 knockdown inhibits HIV-1 replication at the reverse transcription step

In order to address this hypothesis and evaluate the role of RNR2 in HIV-1 replication in MDMs, RNR2 was depleted by the transfection of MDMs with a pool of 4 siRNAs targeting specifically the RNR2 gene and then infected with HIV-1/VSV-G. The control cells were transfected with a pool of non-targeting siRNAs (siCTL). Figure 3A shows that RNR2 siRNAs

(siRNR2) efficiently depleted the protein in US MDMs with respect to control cells (siCTL), as MDM activation by IVIg did. Due to the high sequence homology of RNR2 gene with p53R2 gene (Tanaka, Arakawa et al. 2000), the p53R2 protein expression was also controlled by Western Blot showing that RNR2 siRNAs did not affect p53R2 expression (Figure 3A). mRNA quantification in MDMs from seven different donors confirmed that the increase of p21 expression upon IVIg activation inhibits RNR2 transcription significantly (p=0.0008) (Figure 3B). US MDMs transfection with RNR2 siRNAs reduced RNR2 mRNA by 65 to 98% (p=0.0008) (Figure 3B). Conversely, the levels of p21 mRNA were not significantly affected by RNR2 knockdown in the MDMs (Figure 3 B). After MDM infection with HIV-1/VSV-G, RNR2 knockdown (siRNR2) in US MDMs significantly (p=0.0008) inhibited HIV-1 replication in the seven donors (50 to 98% inhibition) as compared to control US MDM (siCTL) (Figure 3C) indicating that RNR2 is required for HIV-1 replication in MDMs. IVIg activation of MDMs in parallel experiments inhibited HIV-1 replication by similar extent (64 to 94%) (Figure 34C). HIV-1 late reverse transcription products (LRTs) were quantified by qRT-PCR in MDMs from six of the seven donors. RNR2 knockdown (siRNR2) decreased significantly (p=0.002) HIV-1 reverse transcription products by 80 to 93% in the US MDMs (Figure 3D) accordingly to the inhibition of luciferase activity. In the IVIg MDMs similar reduction of HIV-1 reverse transcription products was achieved (70-80%) (Figure 3D).

In conclusion, RNR2 knockdown alone was sufficient to inhibit the reverse transcription step indicating that the RNR2 is an essential HIV-1 co-factor in MDMs that promotes reverse transcription in MDMs.

### p21 inhibits HIV-1 reverse transcription through the down-regulation of RNR2

We then assessed whether p21 inhibits HIV-1 reverse transcription in US MDM and in IVIg MDMs through the down-regulation of RNR2 expression. To this aim US and IVIg MDMs were either silenced for p21 or simultaneously co-silenced for p21 and for RNR2 through the transfection of their respective specific siRNAs (sip21 + siRNR2) and then infected with HIV-1/VSV-G. Control MDMs were transfected with equal amounts of non-targeting siRNAs pools. The p21 and RNR2 expressions were determined through mRNA quantification by qRT-PCR. The use of different amounts of the siCTLs (50 or 100 nM) did affect neither p21 and RNR2 expressions nor the HIV-1 replication. In Figure 4 are represented the results obtained with MDMs from two different donors where the silencing of RNR2 in p21 knockdown cells was successfully obtained. In spite to quantitative variations, the results were consistent between the donors. As seen before, the induction of p21 expression in the IVIg MDMs went with a repression of the RNR2 transcription and reciprocally p21 knockdown (sip21) up-regulated RNR2 expression in both US and IVIg MDMs (Figure 4A). Whereas silencing p21 alone increased or rescued HIV-1 replication (Figures 4B and 4C), HIV-1 replication and reverse transcription remained inhibited in US and IVIg MDMs from both donors simultaneously co-silenced for p21 and RNR2 (sip21 + siRNR2) (Figures 4A, 4B and 4C). These data confirm that p21 inhibits HIV-1 reverse transcription through the down-regulation of RNR2 expression.

### The p21 mediated mechanisms of inhibition of HIV-1 and related-lentiviruses are unrelated to SAMHD1 restriction mechanisms

SAMHD1 was recently demonstrated to restrict HIV-1 replication in macrophages by depleting the intracellular pool of dNTPs (Hrecka, Hao et al. 2011; Laguette, Sobhian et al. 2011; Lahouassa, Daddacha et al. 2012). Moreover, SAMHD1 has been shown to be degraded by HIV-2 and SIV Vpx, which confers these two viruses the ability to escape the SAMHD1 restriction in macrophages and dendritic cells. Although we have previously demonstrated that p21 inhibits HIV-2 and SIV (Bergamaschi, David et al. 2009), we wanted to verify that the p21-mediated mechanisms of HIV-1 reverse transcription described here are also involved in the inhibition of the related-lentiviruses expressing Vpx. We first controlled the expression of SAMHD1 by WB in unstimulated and IVIg activated MDMs. As shown in Figure 5A the induction of p21 protein in IVIg stimulated MDM did not affect SAMHD1 protein levels comparing to unstimulated MDM.Unstimulated (US) MDMs were then silenced for RNR2 through the transfection of RNR2 siRNAs (siRNR2) and infected with SIVmac251 vpx+. The control MDMs were transfected with non-targeting siRNAs pool (siCTL). The IVIg activated MDMs were also infected with SIVmac251. The RNR2 mRNA, quantified by the qRT-PCR, was reduced as expected in the IVIg stimulated MDMs and in the US MDMs silenced for RNR2 (siRNR2) comparing to control MDMs (siCTL) (Figure 5D). The replication of SIVmac251, determined by the quantification of the p27 antigen at 7 days post-infection, was inhibited in the IVIg MDMs (Figure 5D) confirming the previous results (Bergamaschi, David et al. 2009). Importantly, the SIV-mac251 replication was significantly inhibited in the RNR2 knockdown MDMs (siRNR2) (Figure 5D). Figure 5E shows that the SIV-mac251 LRTs, quantified by the qRT-PCR, were strongly decreased (94%) in the RNR2 knockdown MDMs (siRNR2) indicating that RNR2 is an essential cellular co-factor for SIVmac251 reverse transcription in MDMs. As conclusion, RNR2 knockdown is able to inhibit SIV replication at the same extent as HIV-1 indicating that the p21-mediated mechanisms of inhibition of HIV-1 and related-lentiviruses are both related to the same mechanism and un-related to the SAMHD1 restriction pathways.

### p21 represses RNR2 transcription through the down-regulation of E2F1

The induction of RNR2 transcription has been shown to depend on the transcription factor E2F1 in fibroblast cell lines (DeGregori, Kowalik et al. 1995; DeGregori, Leone et al. 1997; Ishida, Huang et al. 2001). In order to assess whether p21 is repressing RNR2 gene expression through the regulation of E2F1 in MDMs, we first studied the effect of the induction of p21 by IVIg stimulation or siRNA-mediated p21 knockdown (sip21) on E2F1 protein and mRNA levels, by Western Blot and qRT-PCR respectively. E2F1 protein band was very faint in US MDMs but was undetectable after p21 induction by IVIg (Figure 6A). Importantly, when p21 was knocked down, the E2F1 protein level was strongly increased indicating that p21 is inhibiting the E2F1 expression (Figure 6A). The quantification of E2F1 mRNA in MDMs from four different donors shows that, similarly to the RNR2 mRNA, E2F1 mRNA was reduced by 65% to 80% (p=0.014) along with the increase of p21 expression while is up-regulated by 2,15 to 8 fold after p21 depletion (p=0.014) (Figure 6B). p21 knockdown, increased E2F1 mRNA levels 1.33- to 4.37- fold (p=0.014). In IVIg activated MDM (Figure 6B). These data demonstrate that p21 is repressing the E2F1 transcription. To assess the effect of E2F1 on the RNR2 transcription in MDMs, we then silenced E2F1 through the transfection of a pool of four specific siRNAs (siE2F1) and the RNR2 and p21 mRNAs were quantified by qRT-PCR. In E2F1 silenced MDMs (siE2F1) from four different donors the E2F1 mRNA was reduced by 50 to 80% compared to control cells transfected with non-targeting siRNAs pool (siCTL) (Figure 6C) (p=0.014). Importantly, in E2F1 knocked down MDM RNR2 mRNA was decreased by 30 to 70% (p=0.014) while the p21 mRNA was unchanged indicating that E2F1 specifically trans-activates RNR2 transcription in MDMs (Figure 6C). Finally, we explored whether the E2F1 knockdown gives the same phenotype of HIV-1 replication and reverse transcription inhibition as observed after RNR2 knockdown. The replication of HIV-1/VSVG in MDMs silenced for E2F1 (siE2F1) from four different donors was inhibited by 73 to 92% (p=0.014) (Figure 6D). Accordingly, quantification of the HIV-1 LRTs in MDMs from three of these four donors showed that the E2F1 knockdown inhibited reverse transcription by 56 to 87% (p=0.03) (Figure 6E) indicating that E2F1 is a cellular co-factor that promotes HIV-1 reverse transcription in MDMs through the transactivation of RNR2 expression.

Altogether these data demonstrate that p21 inhibits HIV-1 reverse transcription through the repression of E2F1 transcription which successively results in the down-regulation of RNR2 gene expression which causes the blocking of the dNTP biosynthesis and reduces the availability of dNTPs for HIV-1 cDNA synthesis.

### p21 down-regulation of E2F1 and restriction of HIV-1 in MDM is independent of RB

It has been described that p21 can repress the transcription of E2F1 target genes through a well-established mechanism in which p21, by inhibiting the CDKs (Cyclin-Dependant protein kinases), maintains RB (retinoblastoma tumor repression protein) in the activated under-phosphorylated state able to retain E2F1 and to inhibit its transactivation activity (Dotto 2000; Perkins 2002). p21 has been shown to bind E2F1 through its N-terminal region (1-90 aa) (Delavaine and La Thangue 1999) and p21 is also able to inhibit E2F1 activity directly and independently from RB (Dimri, Nakanishi et al. 1996; Delavaine and La Thangue 1999). Therefore we wanted to understand whether RB is involved or not in p21-mediated suppression of E2F1 that we have observed in MDMs. Moreover, we wanted to investigate the potential role of RB in the mechanism of p21-mediated restriction of HIV-1 reverse transcription because activated RB has been shown to regulate gene expression of dNTPs biosynthesis enzymes in cell lines (Angus, Wheeler et al. 2002). To this aim the RB mRNA was first quantified by qRT-PCR following the up-regulation or the knockdown of p21 in MDMs. Results from three different healthy donors showed that the RB mRNA was not affected significantly following the increase or the decrease of p21 expression (Figure 7A). Among seven donors analyzed by mRNA quantification or Western Blot the RB expression was reduced modestly (20-40%) in the IVIg MDMs from only three donors (see for example donor in Fig.7C). Since the transcription repression activity of RB depends on its phosphorylation state (Dotto 2000; Perkins 2002), the absence of the regulation of its expression by p21 does not rule out a role for RB in p21-mediated HIV-1 restriction. Therefore, we silenced RB in MDMs to assess whether its depletion is able to rescue HIV-1 replication similarly to the effect of p21 depletion. The silencing of RB was performed through the transfection of a pool of four siRNAs specific to RB gene (siRB1). The control MDMs were transfected with a pool of non-targeting siRNAs (siCTL) and in parallel p21 was also knocked down (sip21) in the IVIg MDMs to compare the effects of the two factors on HIV-1 replication. RB siRNAs transfection efficiently silenced RB in MDMs (70%) and IVIg-activated MDM (85%)as verified by Western Blot (Figure 7B) and mRNA quantification (Figure78C). Importantly, in contrast to p21 knockdown, RB silencing did not modify either E2F1 or RNR2 gene expressions (Figure 7C) indicating that in MDMs, differently from what described in cell lines (Angus, Wheeler et al. 2002), RB did not affect the transcription of RNR2. Consistently, RB knockdown was not able to increase HIV-1 replication in US MDMs or to rescue HIV-1 replication in IVIg MDMs differently from p21 silencing (Figure 7D). These data rule out a determinant contribution of RB in p21-mediated restriction of HIV-1 replication and suggest a direct modulation of E2F1 expression by p21.

### DISCUSSION

Macrophages are target cells for HIV-1 and play a crucial role in the establishment of viral reservoirs and in pathogenesis. We have previously identified p21 as a limiting factor of HIV-1 and related primate lentiviruses in macrophages and showed that its induction by different stimuli, including FcγR aggregation, blocks the accumulation of viral reverse transcripts and eventually suppresses viral integration (Bergamaschi, David et al. 2009). Here we unraveled the mechanism underlying p21-mediated HIV-1 inhibition in macrophages: we show for the first time that p21 causes the reduction of cellular dNTP pools that are needed for HIV reverse transcription, by suppressing the expression of the RNR subunit RNR2 through the down-regulation of the transcription factor E2F1. Therefore, the mechanism of p21-mediated lentiviral restriction affects the synthesis of dNTP and differs from SAMHD1 catabolic pathway.

Lentiviral reverse transcription in non-cycling cells such as macrophages is much slower than in activated CD4+ T cells (O'Brien, Namazi et al. 1994). A rate-limiting step in viral cDNA synthesis in non-dividing cells is the low content of cellular dNTP that are approximately 200 fold lower than in activated T cells(Diamond, Roshal et al. 2004). Although lentiviral reverse transcriptase has acquired the ability to catalyze DNA synthesis in conditions of limited dNTP levels (Diamond, Roshal et al. 2004), it still needs dNTP above a critic threshold. Indeed, dNTP degradation by the cellular DNA phosphorylase SAMHD1 impairs HIV-1 reverse transcription in non-cycling myeloid cells, such as dendritic cells, macrophages and resting CD4 T cells (Hrecka, Hao et al. 2011; Laguette, Sobhian et al. 2011; Baldauf, Pan et al. 2012; Descours, Cribier et al. 2012) by decreasing dNTPs to levels insufficient to an efficient reverse transcriptase processivity (Hrecka, Hao et al. 2011; Laguette, Sobhian et al. 2011; Lahouassa, Daddacha et al. 2012). SIV and HIV-2 counteract SAMHD1 by the protein Vpx that triggers SAMHD1 proteosomal degradation (Hrecka, Hao et al. 2011; Laguette, Sobhian et al. 2011). The inventors show here that p21 restricts reverse transcription of HIV-1 and HIV related lentiviruses such as SIV by affecting the *de novo* synthesis of dNTP, through the suppression of a key enzyme of this pathway, RNR2. In mammalian cells, dNTP synthesis and degradation pathways are finely tuned to ensure the balance of the four dNTP pools (Rampazzo, Miazzi et al. 2010). The predominant pathway to dNTP synthesis requires the reduction of ribonucleoside diphosphates to the corresponding deoxynucleoside diphosphates that are then phosphorylated to triphosphates. The de novo synthesis of dNTP is thightly regulated by the regulation of the enzyme ribonucleotide reductase (RNR) (Thelander and Reichard 1979; Nordlund and Reichard 2006). In mammalian cells RNR consists of two non-identical subunits: the RNR1 large subunit contains binding sites for catalysis and regulation. The RNR2 small subunit provides a tyrosyl free radical essential for nucleotide reduction. Besides RNR2 another small subunit homologous, p53R2, has been described (Tanaka, Arakawa et al. 2000). So far, the regulation of RNR activity has been studied in proliferating and quiescent cell lines, mostly of murine origin, and never in terminally differentiated primary cells including macrophages. In cycling cells, RNR is a complex between RNR1 and RNR2. RNR2 is degraded during late mitosis (Chabes, PNAS, 2003). Quiescent fibroblast cell lines contain p53R2 and little or no RNR2 (Bjorklund, Skog et al. 1990; Hakansson, Hofer et al. 2006). Therefore it is thought that p53R2 together with RNR1 supports *de novo* synthesis of dNTPs for basal DNA repair in non-cycling cells that contain insufficient RNR2. However, recent reports suggest that p53R2 in quiescent cells is mainly required for mitochondrial DNA replication and repair (Bourdon, Minai et al. 2007; Pontarin, Ferraro et al. 2012).

We easily detected both RNR2 transcripts and protein in un-stimulated MDM that do not cycle. Some reports described minor subpopulations of monocytes that re-enter the cell cycle after treatment with macrophage- and granulocyte macrophage-colony stimulating factors (GM-CSF) (Mock, Hollenbaugh et al 2012). In our cultures, monocytes are not differentiated using these cytokines. Moreover, cell cycle analysis by propidium iodide showed that MDM were all in G0/G1 phase. The detection of RNR2 expression in MDM, although at low levels, suggests that this subunit is active in the RNR complex and contributes to the synthesis of dNTPs for DNA repair in these cells. Importantly, RNR2 suppression through p21 induction and by siRNAs mediated knockdown was sufficient to block the synthesis of HIV-1 cDNA by reverse transcriptase, indicating that RNR2 is a necessary cofactor of HIV-1 replication in macrophages. Accordingly, an increase in RNR2 concentrations in macrophages differentiated upon Leihsmania infection has been associated with an enhancement of HIV-1 replication (Mock, Hollenbaugh et al. 2012).

Our data indicate that p21 regulates RNR2 expression in MDM at transcriptional level, in agreement with previous studies performed on a p21 inducible fibrosarcoma cell lines (Chang, Watanabe et al. 2000; Angus, Wheeler et al. 2002; Broude, Swift et al. 2007). However, in contrast to these cell lines, we found that p21 did not modulate the RNR1 expression in macrophages. As p21 does not contain any putative DNA-binding domain, the interaction with RNR2 promoter must be indirect, i.e., through another transcription factor or co-factor. RNR2 promoter contains E2F elements (Chaboute, Clement et al. 2000; Pham, Kos et al. 2006) and RNR2 transcription in fibroblast cell lines is dependent on the transcription factor E2F1 (DeGregori, Kowalik et al. 1995; DeGregori, Leone et al. 1997; Ishida, Huang et al. 2001). p21 has been implicated in the repression of gene transcription through the control of E2F transcription factors (DeGregori, Kowalik et al. 1995; DeGregori, Leone et al. 1997; Ishida, Huang et al. 2001). p21 can control E2F transcriptional activity through two different pathways : one implicating RB (Angus, Wheeler et al. 2002) and another independent of RB acting through a direct interaction between p21 and E2F1 (Dimri, Nakanishi et al. 1996; Delavaine and La Thangue 1999). The RB-sensitive E2F activity is induced late in G1 in proliferating cells (Dimri, Nakanishi et al. 1996; Verona, Moberg et al. 1997). p21 maintains RB in the active hypo-phosphorylated form, which negatively regulates the activity of E2F transcription factors, by the inhibition of CDK2 kinase during the transition through G1 in cycling cells. Accordingly, RB has been shown to down-regulate the transcription of several genes involved in dNTP synthesis and consequently to reduce dNTP pools in rat cell lines (Angus, Wheeler et al. 2002). However, studies on p21 impact on E2F1 transcriptional activity have been essentially related to p21 activity in cell growth inhibition in proliferating cell lines and are not directly transposable to macrophages. Our results show that RB silencing does not significantly affect RNR2 expression or HIV-1 replication in macrophages. These data strongly suggest that RB is not involved in p21-mediated RNR2 regulation in macrophages and rule out a determinant role for RB in p21-mediated HIV-1 restriction.

In contrast with the weak or no effects on RB, the depletion of p21 in MDM strongly induced the expression of E2F1 transcripts and protein, the increase in E2F1 paralleling that of RNR2. More important, siRNA-mediated depletion of E2F1 down-regulated RNR2 expression and blocked HIV-1 replication by decreasing HIV-1 reverse transcripts. Thus reproducing the phenotype of restriction associated to p21 induction by FcγR aggregation.

These data strongly support the following mechanism (Figure 8): p21 regulates E2F1 expression by interaction with E2F1; p21 induction strongly down-regulates E2F1 transcription, possibly blocking its auto-activation from the E2F-binding sites in the promoter(Johnson, Ohtani et al. 1994; Neuman, Flemington et al. 1994). The inhibition of E2F1 expression by p21 is followed by the suppression of RNR2 transcription that blocks the *de novo* synthesis of dNTP. Continuous dNTP degradation by cellular enzymes in the absence of dNTP synthesis leads to the decrease of dNTP pool available for HIV-1 cDNA synthesis impairing the reverse transcription process.

It is likely that this mechanism affects HIV-1 reverse transcription specifically in cells, such as macrophages and dendritic cells, with limited dNTP resources. Interestingly, transcriptome analysis showed an up-regulation of p21 and a down-regulation of E2F1 and RNR enzymes, including RNR2, in activated by immune complexes murine dendritic cells (van Montfoort, t Hoen et al. 2012). Whether the p21-E2F1-RNR2 pathway controls HIV replication in human dendritic cells and in hematopoietic stem cells (Zhang, Scadden et al. 2007) remains to be determined.

Besides impairment of HIV cDNA synthesis, the depletion of dNTP pool that follows RNR2 down-regulation may also increase the misincorporation of cellular rNTP that are efficiently incorporated in HIV-1 DNA in macrophages (Ferraro NAR 2010). Since dTTP levels were increased in macrophages upon p21 knockdown, a corresponding reduction of dTTP levels after induction of p21 may also favor the misincorporation of dUTP that is abundant in macrophages in the nascent HIV-1 DNA (Kennedy, Daddacha et al.). Both rNTP and dUTP misincorporations would lead to a reduced processivity of viral reverse transcriptase and to an enhanced degradation of the reverse transcripts.

The absolute requirement of dNTP availability for HIV-1 reverse transcriptase activity has suggested that a RNR inhibitor, hydroxyurea, may be used, in combination with nucleotide analogs, in the treatment of AIDS, to decrease the processivity of HIV reverse transcriptase (Gao, Cara et al. 1993). Our results suggest that dNTP pool size and hence HIV-1 cDNA synthesis may be affected either by targeting directly RNR2 or by inhibiting its expression through p21 or E2F1 targeting. All these molecules are actively studied as targets in cancer therapy. Statins have been shown to have anti-proliferative activity by inducing p21 (Ukomadu and Dutta 2003). p21-dependent inhibition of colon cancer cell growth by mevastatin is independent of inhibition of G1 cyclin-dependent kinases and are being investigated for their anti-tumorigenic activities. Histone deacetylase inhibitors (HDACi) are being investigated as anticancer targets with the aim to reverse aberrant epigenetic modifications associated with cancer (Bolden, Peart et al. 2006). HDACi function, at least partly, through their ability to promote the induction of p21 (Ocker and Schneider-Stock 2007). The inventors have previously shown that treatment of macrophages with HDACi induced p21 and inhibited HIV-1 reverse replication (Bergamaschi, David et al. 2009). Noteworthy, HDACi are also actively studied in AIDS treatment in combination with highly active antiretroviral therapy to eradicate HIV-1 from latent cellular reservoirs (Keedy, Archin et al. 2009). Besides their activity in reactivating latent provirus expression, HDACi might also block HIV-1 replication in newly infected macrophages by inducing p21. Either induction or repression of E2F1 has been proposed to induce cancer cell apoptosis or reducing their metastatic potential respectively (Engelmann and Putzer 2012). However targeting directly E2F1 may be challenging owing to its opposite functions in cell death and proliferation in cycling cells. RNR is an important therapeutic target for cancer therapy: RNR inhibition decreasing dNTP concentration inhibits DNA synthesis and induces cycle arrest and apoptosis of cancer cells. Inhibitors specific that inhibit free radical formation in RNR2, are currently studied as cytostatic agents (Shao, Zhou et al. 2006). Considering the present results, considering the extension of the treatment with RNR2 inhibitors to HIV-1 infected patients, is a valuable approach including in complement to cART.

### MATERIALS AND METHODS

### Monocyte Derived Macrophages (MDMs)

Buffy coats (fraction of anticoagulated blood sample that contains most of white blood cells following gradient density centrifugation) from blood healthy donors were obtained through the French blood bank (Etablissement Francais du sang (EFS)) as part of EFS-Institute Pasteur Convention. Written informed consent was obtained from each donor to use the cells for clinical research in accordance with French law. Monocytes were isolated from buffy coats and differentiated into macrophages as previously described (David, Saez-Cirion et al. 2006; Bergamaschi, David et al. 2009). Briefly, monocytes were separated from peripheral blood mononuclear by adherence to the plastic and then detached and cultured for 6 to 7 days in hydrophobic Teflon dishes (Lumox; Dutsher) in macrophages medium (RPMI 1640 supplemented with 200 mM L-glutamine, 100 U of penicillin, 100 µg streptomycin, 10 mM HEPES, 10 mM sodium pyruvate, 50 µM β-mercaptoethanol, 1% minimum essential medium vitamins, 1% non-essential amino acids) supplemented with 15% of heat inactivated human AB serum. For experiments, MDMs were harvested and resuspended in macrophage medium containing 10% of heat inactivated fetal bovine serum (FBS). MDMs obtained with this method are 91 to 96 % CD14, they express: the Fcγ receptors (CD16, CD32 and CD64), the differentiation markers (C11b and CD71) and the M2 macrophage polarization markers (CD163 and CD206). For Fcγ receptors stimulation, wells of 96-well plate, 24-well plate and 12-well plate were pre-coated for 2 hours at 37 °C with respectively 100 µl, 250 µl and 500 µl of 1 mg/ml of human IgG for therapeutic use (IVIg) (Endobuline; Baxter) in PBS. The inventors have previously shown that immobilized IVIg and preformed immune complexes have the same impact both on the induction of p21 and the inhibition of HIV-1 replication in MDM (David, Saez-Cirion et al. 2006). MDMs (1x10⁶/ml of macrophage medium + 10% FBS) were then seeded on the un-coated or IVIg pre-coated wells (6.5x10⁴ MDMs/well of 96-well plate, 2.5x10⁵ MDMs/ well of 24-well plate and 0.5x10⁶ MDMs/ well of 12-well plate). Unstimulated (US) and IVIg stimulated MDMs were let to be attached on the plates for 2 hours at 37 °C before infections or before siRNAs transfection.

### siRNAs transfection

The small interfering RNAs (siRNAs) were all purchased from Dharmacon. The siRNA against p21 gene (sip21) is on target plus siRNA n.12: 5' AGA CCA GCA UGA CAG AUU U 3'. The siRNAs against RNR2 (siRNR2), E2F1 (siE2F1) and RB (siRB) genes are siGenome smart pool selected composed each of a pool of four siRNAs. The different siRNAs have the following sequences: **siRNR2:** 1- 5' GCA CUC UAA UGA AGC AAU A 3' (SEQ ID No.1), 2- 5' GAA GAG UAG GGA GUA U 3' (SEQ ID No.2), 3- 5' GAG UAG AGA ACC CAU UUG A 3' (SEQ ID No.3) and 4- 5' GGC UCA GCU UGG UCG ACA A 3' (SEQ ID No.4); **siE2F1:** 1- 5' GAA CAG GGC CAC UGA CUC U 3' (SEQ ID No.5), 2- 5' UGG ACC ACC UGA UGA AUA U 3' (SEQ ID No.6), 3- 5' CCC AGG AGG UCA CUU CUG A 3' (SEQ ID No.7) and 4- 5' GGC UGG ACC UGG AAA CUG A 3' (SEQ ID No.8). **siRB:** 1- 3' GAA AGG ACA UGU GAA CUU A 5' (SEQ ID No.9), 2-3'GAA GAA GUA UGA UGU AUU G 5' (SEQ ID No.10), 3- 3' GAA AUG ACU UCU ACU CGA A 5' (SEQ ID No.11) and 4- 3' GGU UCA ACU ACG CGU GUA A 5' (SEQ ID No.12). The control siRNAs are a pool of four on target plus non-targeting siRNAs.

siRNAs transfection was performed using INTERFERin kits (Polyplus Transfection).MDMs (1x10⁶/ml of macrophages medium + 10% FBS) were seeded on un-coated or IVIg pre-coated wells (6.5x10⁴ MDMs/well of 96-well plate, 2.5x10⁵ MDMs/well of 24-well plate and 0.5x10⁶ MDMs/ well of 12-well plate) and let to be attached at 37°C for 2 hours prior siRNAs transfection. The siRNAs transfections were performed with the INTERFERin (Polyplus Transfection). Different amounts of siRNAs were pre-diluted in 1 ml of Opti-MEM in which 20 µl of INTERFERin were added and the transfection mix was let to incubate at room temperature for 10 minutes. The transfection mix volumes 32 µl, 125 µl and 250 µl were added respectively to 6.5x10⁴, 2.5x10⁵ and 0.5x10⁶ MDMs at the final concentrations of 50 nM for sip21, siRNR2 and siE2F1 siRNAs and of 25 nM for siRB1 siRNAs. Equal amounts of the on target plus non-targeting siRNAs were added to the control MDMs. The MDMs were then incubated at 37°C for 22 hours. The medium was replaced with fresh macrophage medium supplemented with 10% FBS prior to the infections. Cell lysates were assayed for protein expression by Western Blot and for the quantification of mRNA by the Real-Time quantitative PCR (RT-qPCR) to determine the efficiency of gene knockdown at the moment of infection. The Western Blot analysis of the RB knockdown MDMs was performed at 44 hours post siRNAs transfection.

### Cell infections

The NL4.3-Luc HIV-1 pseudotyped with VSV-G (HIV-1/VSVS-G) was produced and quantified as previously described (David, Saez-Cirion et al. 2006; Bergamaschi, David et al. 2009). The SIVmac251 replication competent was produced through the infection of CEM x 174 T cell lines. MDMs (1x10⁶/ml of macrophage medium + 10% FBS) were seeded either on the un-coated or IVIg coated wells (6.5x10⁴ MDMs/well of 96-well plate, 2.5x10⁵ MDMs/ well of 24-well plate and 0.5x10⁶ MDMs/ well of 12-well plate). MDMs were let to be attached on the plate for 2 hours at 37°C prior to the infections. For knockdown cells the infections were performed 24 hours post IVIg stimulation and 22 hours post siRNAs transfections. MDMs were infected with 4 - 5 ng of p24 of HIV-1/VSV-G or 300 ng p27 of SIV-mac 251 for 1x10⁶ cells by 1 hour of spinoculation (1,200 g at room temperature) followed by 1 hour at 37°C. Cells were then washed with PBS and incubated at 37°C in fresh macrophage medium supplemented with 10% FBS for 72 hours for HIV-1 replication determined by luciferase activity and LRTs (Late Reverse Transcripts) quantification by qRT-PCR. For SIVmac251 replication the culture supernatants were harvest 7 days post infection and quantified for their p27 antigen content using the SIV p27 ELISA kit (Zeptometrix Crop). The SIV LRTs quantification was performed at 48 hours post infection. In the experiments for viral cDNA detection with qRT-PCR, the viral stocks were pretreated with DNase I (Roche Diagnostics) for 1 hour at room temperature. For the addition of the deoxynucleosides (dNs), the un-stimulated (US) and IVIg stimulated MDMs were treated with the indicated concentrations of dNs immediately after infection for 24 hours at 37 °C. The deoxynucleosides consisted of mixture of dA (D8668), dC (D0776), dG (D0901) and dT (T1895; all from Sigma Aldrich).

### Quantification of the dNTPs pools

The dNTPs were quantified as previously described (Diamond, Roshal et al. 2004). The p21 knockdown (sip21) and control (siCTL) MDMs (3x10⁶ MDMs each) were washed with cold PBS and lyzed, at 22 hours post-siRNAs transfection, in 2 ml of 60% (vol/vol) of aqueous methanol. Lysates were heated at 95 °C for 7 minutes, clarified by centrifugation at 16,000 g for 15 minutes at 4 °C, dried under vacuum. Extracted dNTPs were then resuspended and quantified as previously described (Diamond, T. L., 2004).

### Quantitative Real Time PCR analysis: qRT-PCR

For the mRNA quantification, total RNA from 2.5x10⁵ to 0.5x10⁶ MDMs was extracted with the RNeasy kit (Qiagen) and treated with DNase according to the manufacturer's instructions. The RNA was transcribed with the SuperScript II RT (Invitrogen). PCR amplifications of cDNA was carried out in duplicate in MicroAmp Optical 96-well reaction plate (30 µl/well), using 5 µl cDNA template, 15 µl of 2x Taqman Universal Master mix (Applied biosystems), 1.5 µl of Taqman gene expression premade mix (Applied biosystems) (GAPDH: Hs99999905_m1; RNR2: Hs00357247_g1; p21: Hs00355782; RB: Hs01078066_m1; E2F1: Hs00153451_m1) and completed with H₂O up to 30 µl. The amplification conditions were: 50°C for 2 min and 95°C for 10 min followed by 40 cycles at 95°C for 15 s and 60°C for 1 min on ABI prism 7000 Sequence Detection System. The data were analyzed with the cycle threshold (C_{T}) method, and the amount of target mRNA in each sample was normalized to GAPDH mRNA as an endogenous reference.

For the quantification of the HIV-1 Late Reverse Transcripts (LRTs), the MDMs DNA was extracted with the DNeasy Tissue Kit (Qiagen) at 72 hours post-infection. The qRT-PCR analysis of LRTs through the quantification of the U5-Gag viral DNA were carried on an ABI prism 7000 Sequence Detection System as previously described (David, Saez-Cirion et al. 2006; Bergamaschi, David et al. 2009). Standards for U5-Gag amplification products were generated by serial dilution of DNA extracted from HIV-1 8E5 cells containing one integrated copy of HIV-1 per cell. The quantification of the SIVmac251 LRTs was performed as previously described (Hofmann-Lehmann, Swenerton et al. 2000). The amounts of LRTs were normalized to the endogenous reference gene albumin. Standard curve were generated by serial dilution of a commercial human genomic DNA (Roche).

### Bioluminescence quantification in living cells

The technique for quantification of bioluminescence in infected cells has been described in detail elsewhere (Saez-Cirion, A.et al 2004). Briefly, the system includes a fully automated inverted microscope (200M, Carl Zeiss, Germany) and is housed in a light-tight dark box. Low level light emission was collected using an Imaging Photon Detector (IPD 3, Photek Ltd. East Sussex, UK) and brightfield images were obtained with a CCD camera (Coolsnap HQ, Roper Scientific). Observations were made with Plan-Neofluar 10X (air, NA=0.5), 25X (oil, NA=0.8) objectives (Carl Zeiss, Germany). The data acquisition software allowed superposition of photon events with brightfield images and selective quantification of photons in delimited surfaces. 10⁵ MDM were plated after infection on glass-bottomed dishes (MatTek corp, MA, USA), washed and cultured in complete medium for 5 days before analysis. The analyses were conducted in PBS + 5% FCS or complete phenol red free medium at room temperature in presence of endotoxin free beetle D-luciferin (Promega) (final concentration 1 mM).

### Western Blot analysis and antibodies

MDMs (0.5x10⁶) were lyzed in 100 µl of lysis buffer (50 mM Tris-HCl pH 7.4, 150 mM NaCl, 1mM EDTA and 1% Triton X-100) provided with protease and phosphatase cocktails inhibitors (Roche). Protein were quantified with Bradford Kit (Biorad) and 30 to 50 µg of cell extracts were diluted in the Leammli buffer, boiled for 5 min at 95 °C and loaded on the mini-protean-TGX 4-15 % precast acrylamide gel (Biorad) for electrophoresis separation. Proteins were then blotted on the PVDF transfer membrane (GE healthcare). After blocking with 5% skimmed milk, the membrane were incubated with the primary antibodies followed by the secondary horseradish peroxidase anti-goat or anti-rabbit antibodies (Sigma) or anti-mouse (Cell Signaling). The proteins were revealed on hyperfilms (Amersham) by using Super Signal West pico chemiluminescent substrate (Pierce). The primary anti-PCNA (PC10), ant-RNR2 (N-18), anti-E2F1 (KH95), anti-RB (IF8) and anti-RNR1 (H-300) antibodies were all purchased from Santa Cruz and all used at the dilutions 1:200 in 1% skimmed milk in PBS-0.05 % tween. The primary anti-p53R2 (1:500) and anti-GAPDH (1:1000) antibodies were obtained from Abcam. The anti-p21 (1:1000) is from Millipore and the anti-Actin (1:5000) is from Sigma.

### Statistical analysis

Differences among groups were analyzed with the Kruskal-Wallis test (nonparametric test). When a significant difference was detected among the different groups, two-by-two comparisons were performed, applying the Bonferroni correction. Data were analyzed with STATA software version 12.0 (Stata Corporation, College Station, Texas).

### II. RNR2 inhibitors block HIV-1 infection in macrophages.

The inventors assayed RNR2 inhibitors for their effect on reverse transcription in macrophages and especially tested dose dependent inhibition of HIV-1 replication by RNR2 inhibitors consisting of hydroxyurea (HU) or Deferoxamine Mesylate (DFO). Monocyte-derived-macrophages were infected with HIV-1/VSV-G and then treated with decreasing concentrations of hydroxyurea (HU) (40 to 4 mM) (A) or Deferoxamine Mesylate (DFO) (250 to 100 µM) (B). HIV-1 replication was determined by measuring luciferase activity 72 hours post infection. Results are expressed as fold changes of luciferase activity in drug-treated cells with respect to mock treated cells. The data are means ± standard deviation of triplicate wells.

Similar results were obtained with MDM from another donor.

MDM viability was not affected at the concentrations of HU and DFO used in the assays (not shown).

It is observed that concentrations inferior to those usually known to have a toxic effect on cells were suitable to obtain efficiency on reverse transcription inhibition in the assay. The reason might be that dNTP pool size in macrophages is hundreds times lower than in cycling cells (Diamond TL et al, 2013). RNR2 levels are also extremely decreased in macrophages in comparison with cycling cells. Consequently, the concentrations of RNR2 inhibitors required for blocking HIV-1 infection in macrophages should be lower than those currently known to be needed for anti-tumor activity. Therefore, should RNR2 inhibitors be included in antiretroviral therapy, their potential toxic side effects will also be decreased.

### REFERENCES

Ancuta, P., K. J. Kunstman, et al. (2006). "CD16+ monocytes exposed to HIV promote highly efficient viral replication upon differentiation into macrophages and interaction with T cells." Virology 344(2): 267-76.
Angus, S. P., L. J. Wheeler, et al. (2002). "Retinoblastoma tumor suppressor targets dNTP metabolism to regulate DNA replication." J Biol Chem 277(46): 44376-84.
Aquaro, S., P. Bagnarelli, et al. (2002). "Long-term survival and virus production in human primary macrophages infected by human immunodeficiency virus." J Med Virol 68(4): 479-88.
Asada M, Y. T., Ichijo H, Delia D, Miyazono K, Fukumuro K, Mizutani S (1999). "Apoptosis inhibitory activity of cytoplasmic p21(Cip1/WAF1) in monocytic differentiation." EMBO J 18: 1223-1234.
Baldauf, H. M., X. Pan, et al. (2012). "SAMHD1 restricts HIV-1 infection in resting CD4(+) T cells." Nat Med 18(11): 1682-9.
Bergamaschi, A., A. David, et al. (2009). "The CDK inhibitor p21Cip1/WAF1 is induced by FcgammaR activation and restricts the replication of human immunodeficiency virus type 1 and related primate lentiviruses in human macrophages." J Virol 83(23): 12253-65.
Bergamaschi, A., A. David, et al. (2009). "The CDK inhibitor p21Cip1/WAF1 is induced by Fc 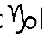 R activation and restricts the replication of HIV-1 and related primate lentiviruses in human macrophages " J Virol **In press.**
Bergamaschi, A. and G. Pancino (2010). "Host hindrance to HIV-1 replication in monocytes and macrophages." Retrovirology 7: 31.
Berger, G., S. Durand, et al. (2011). "APOBEC3A is a specific inhibitor of the early phases of HIV-1 infection in myeloid cells." PLoS Pathog 7(9): e1002221.
Bjorklund, S., S. Skog, et al. (1990). "S-phase-specific expression of mammalian ribonucleotide reductase R1 and R2 subunit mRNAs." Biochemistry 29(23): 5452-8.
Bolden, J. E., M. J. Peart, et al. (2006). "Anticancer activities of histone deacetylase inhibitors." Nat Rev Drug Discov 5(9): 769-84.
Bourdon, A., L. Minai, et al. (2007). "Mutation of RRM2B, encoding p53-controlled ribonucleotide reductase (p53R2), causes severe mitochondrial DNA depletion." Nat Genet 39(6): 776-80.
Broude, E. V., M. E. Swift, et al. (2007). "p21(Waf1/Cip1/Sdi1) mediates retinoblastoma protein degradation." Oncogene 26(48): 6954-8.
Cassol, E., L. Cassetta, et al. (2010). "Macrophage polarization and HIV-1 infection." J Leukoc Biol 87(4): 599-608.
Chaboute, M. E., B. Clement, et al. (2000). "Cell cycle regulation of the tobacco ribonucleotide reductase small subunit gene is mediated by E2F-like elements." Plant Cell 12(10): 1987-2000.
Chang, B. D., K. Watanabe, et al. (2000). "Effects of p21Waf1/Cip1/Sdi1 on cellular gene expression: implications for carcinogenesis, senescence, and age-related diseases." Proc Natl Acad Sci U S A 97(8): 4291-6.
Chen, J., P. K. Jackson, et al. (1995). "Separate domains of p21 involved in the inhibition of Cdk kinase and PCNA." Nature 374(6520): 386-8.
Cobos-Jimenez, V., T. Booiman, et al. (2011). "Macrophages and HIV-1." Curr Opin HIV AIDS 6(5): 385-90.
Collin, M. and S. Gordon (1994). "The kinetics of human immunodeficiency virus reverse transcription are slower in primary human macrophages than in a lymphoid cell line." Virology 200(1): 114-20.
Coqueret O (2003). "New roles for p21 and p27 cell-cycle inhibitors: a function for each cell compartment?" Trends Cell Biol 13: 65-70.
Crowe S, Zhu T, Muller WA: The contribution of monocyte infection and trafficking to viral persistence, and maintenance of the viral reservoir in HIV infection. J Leuk Biol 2003, 74:635-641)
David, A., A. Saez-Cirion, et al. (2006). "The engagement of activating FcgammaRs inhibits primate lentivirus replication in human macrophages." J Immunol 177(9): 6291-300.
DeGregori, J., T. Kowalik, et al. (1995). "Cellular targets for activation by the E2F1 transcription factor include DNA synthesis- and G1/S-regulatory genes." Mol Cell Biol 15(8): 4215-24.
DeGregori, J., G. Leone, et al. (1997). "Distinct roles for E2F proteins in cell growth control and apoptosis." Proc Natl Acad Sci U S A 94(14): 7245-50.
Delavaine, L. and N. B. La Thangue (1999). "Control of E2F activity by p21Waf1/Cip1." Oncogene 18(39): 5381-92.
Descours, B., A. Cribier, et al. (2012). "SAMHD1 restricts HIV-1 reverse transcription in quiescent CD4+ T-cells." Retrovirology 9: 87.
Diamond, T. L., M. Roshal, et al. (2004). "Macrophage tropism of HIV-1 depends on efficient cellular dNTP utilization by reverse transcriptase." J Biol Chem 279(49): 51545-53.
Diamond TL et al, J Biol Chem 2004; Amie SM et al Virology, 2013
Dimri, G. P., M. Nakanishi, et al. (1996). "Inhibition of E2F activity by the cyclin-dependent protein kinase inhibitor p21 in cells expressing or lacking a functional retinoblastoma protein." Mol Cell Biol 16(6): 2987-97.
Dotto, G. P. (2000). "p21(WAF1/Cip1): more than a break to the cell cycle?" Biochim Biophys Acta 1471(1): M43-56.
Engelmann, D. and B. M. Putzer (2012). "The dark side of E2F1: in transit beyond apoptosis." Cancer Res 72(3): 571-5.
Furge, L. L. and F. P. Guengerich (1997). "Analysis of nucleotide insertion and extension at 8-oxo-7,8-dihydroguanine by replicative T7 polymerase exo- and human immunodeficiency virus-1 reverse transcriptase using steady-state and pre-steady-state kinetics." Biochemistry 36(21): 6475-87.
Gao, W. Y., A. Cara, et al. (1993). "Low levels of deoxynucleotides in peripheral blood lymphocytes: a strategy to inhibit human immunodeficiency virus type 1 replication." Proc Natl Acad Sci U S A 90(19): 8925-8.
Gartner, S., P. Markovits, et al. (1986). "The role of mononuclear phagocytes in HTLV-III/LAV infection." Science 233(4760): 215-9.
Goldstone, D. C., V. Ennis-Adeniran, et al. (2011). "HIV-1 restriction factor SAMHD1 is a deoxynucleoside triphosphate triphosphohydrolase." Nature 480(7377): 379-82.
Gu Y, T. C., Morgan DO (1993). "Inhibition of CDK2 activity in vivo by an associated 20K regulatory subunit." Nature 366: 707-710.
Hakansson, P., A. Hofer, et al. (2006). "Regulation of mammalian ribonucleotide reduction and dNTP pools after DNA damage and in resting cells." J Biol Chem 281(12): 7834-41.
Harper JW, A. G., Wei N, Keyomarsi K, Elledge SJ (1993). "The p21 Cdk-interacting protein Cip1 is a potent inhibitor of G1 cyclin-dependent kinases." Cell 75: 805-816.
Herbein, G., A. Coaquette, et al. (2002). "Macrophage activation and HIV infection: can the Trojan horse turn into a fortress?" Curr Mol Med 2(8): 723-38.
Hofmann-Lehmann, R., R. K. Swenerton, et al. (2000). "Sensitive and robust one-tube real-time reverse transcriptase-polymerase chain reaction to quantify SIV RNA load: comparison of one- versus two-enzyme systems." AIDS Res Hum Retroviruses 16(13): 1247-57.
Hrecka, K., C. Hao, et al. (2011). "Vpx relieves inhibition of HIV-1 infection of macrophages mediated by the SAMHD1 protein." Nature 474(7353): 658-61.
Ishida, S., E. Huang, et al. (2001). "Role for E2F in control of both DNA replication and mitotic functions as revealed from DNA microarray analysis." Mol Cell Biol 21(14): 4684-99.
Jamburuthugoda, V. K., P. Chugh, et al. (2006). "Modification of human immunodeficiency virus type 1 reverse transcriptase to target cells with elevated cellular dNTP concentrations." J Biol Chem 281(19): 13388-95.
Johnson, D. G., K. Ohtani, et al. (1994). "Autoregulatory control of E2F1 expression in response to positive and negative regulators of cell cycle progression." Genes Dev 8(13): 1514-25.
Kedzierska, K., R. Azzam, et al. (2003). "Defective phagocytosis by human monocyte/macrophages following HIV-1 infection: underlying mechanisms and modulation by adjunctive cytokine therapy." J Clin Virol 26(2): 247-63.
Keedy, K. S., N. M. Archin, et al. (2009). "A limited group of class I histone deacetylases acts to repress human immunodeficiency virus type 1 expression." J Virol 83(10): 4749-56.
Kennedy, E. M., W. Daddacha, et al. "Abundant non-canonical dUTP found in primary human macrophages drives its frequent incorporation by HIV-1 reverse transcriptase." J Biol Chem 286(28): 25047-55.
Koenig, S., H. E. Gendelman, et al. (1986). "Detection of AIDS virus in macrophages in brain tissue from AIDS patients with encephalopathy." Science 233(4768): 1089-93.
Laguette, N., B. Sobhian, et al. (2011). "SAMHD1 is the dendritic- and myeloid-cell-specific HIV-1 restriction factor counteracted by Vpx." Nature 474(7353): 654-7.
Lahouassa, H., W. Daddacha, et al. (2012). "SAMHD1 restricts the replication of human immunodeficiency virus type 1 by depleting the intracellular pool of deoxynucleoside triphosphates." Nature immunology 13(6): 621.
Lahouassa, H., W. Daddacha, et al. (2012). "SAMHD1 restricts the replication of human immunodeficiency virus type 1 by depleting the intracellular pool of deoxynucleoside triphosphates." Nat Immunol 13(3): 223-8.
Lee Y et al Cancer Res (2003). "Biochemical Modulation of Aracytidine (Ara-C) Effects by GTI-2040, a Ribonucleotide Reductase Inhibitor, in K562 Human Leukemia Cells", Jun 1, 63(11): 2802-11
Liu, M., A. lavarone, et al. (1996). "Transcriptional activation of the human p21(WAF1/CIP1) gene by retinoic acid receptor. Correlation with retinoid induction of U937 cell differentiation." J Biol Chem 271(49): 31723-8.
Meyerhans, A., J. P. Vartanian, et al. (1994). "Restriction and enhancement of human immunodeficiency virus type 1 replication by modulation of intracellular deoxynucleoside triphosphate pools." J Virol 68(1): 535-40.
Mock, D. J., J. A. Hollenbaugh, et al. (2012). "Leishmania induces survival, proliferation and elevated cellular dNTP levels in human monocytes promoting acceleration of HIV co-infection." PLoS Pathog 8(4): e1002635.
Neuman, E., E. K. Flemington, et al. (1994). "Transcription of the E2F-1 gene is rendered cell cycle dependent by E2F DNA-binding sites within its promoter." Mol Cell Biol 14(10): 6607-15.
Nordlund, P.et al (1993) "Structure and function of the Escherichia Coli ribonucleotide reductase protein R2" J. Mol. Biol. 232, 123-164
Nordlund, P. and P. Reichard (2006). "Ribonucleotide reductases." Annu Rev Biochem 75: 681-706.
O'Brien, W. A., A. Namazi, et al. (1994). "Kinetics of human immunodeficiency virus type 1 reverse transcription in blood mononuclear phagocytes are slowed by limitations of nucleotide precursors." J Virol 68(2): 1258-63.
Ocker, M. and R. Schneider-Stock (2007). "Histone deacetylase inhibitors: signalling towards p21cip1/waf1." Int J Biochem Cell Biol 39(7-8): 1367-74.
Orenstein, J. M., C. Fox, et al. (1997). "Macrophages as a source of HIV during opportunistic infections." Science 276(5320): 1857-61.
Peng, G., T. Greenwell-Wild, et al. (2007). "Myeloid differentiation and susceptibility to HIV-1 are linked to APOBEC3 expression." Blood 110(1): 393-400.
Perkins, N. D. (2002). "Not just a CDK inhibitor: regulation of transcription by p21(WAF1/CIP1/SD11)." Cell Cycle 1(1): 39-41.
Pham, D. Q., P. J. Kos, et al. (2006). "Regulation of the ribonucleotide reductase small subunit (R2) in the yellow fever mosquito, Aedes aegypti." Gene 372: 182-90.
Pontarin, G., P. Ferraro, et al. (2012). "Mammalian ribonucleotide reductase subunit p53R2 is required for mitochondrial DNA replication and DNA repair in quiescent cells." Proc Natl Acad Sci U S A 109(33): 13302-7.
Powell, R. D., P. J. Holland, et al. (2011). "Aicardi-Goutieres syndrome gene and HIV-1 restriction factor SAMHD1 is a dGTP-regulated deoxynucleotide triphosphohydrolase." J Biol Chem 286(51): 43596-600.
Rampazzo, C., C. Miazzi, et al. (2010). "Regulation by degradation, a cellular defense against deoxyribonucleotide pool imbalances." Mutat Res 703(1): 2-10.
Saez-Cirion, A., Nicola, M. A., Pancino, G., & Shorte, S. L. (2006) Biotechnology journal 1,682-689.
Shao, J., B. Zhou, et al. (2006). "Ribonucleotide reductase inhibitors and future drug design." Curr Cancer Drug Targets 6(5): 409-31.
Sharova, N., C. Swingler, et al. (2005). "Macrophages archive HIV-1 virions for dissemination in trans." Embo J 24(13): 2481-9.
Shen, R., H. E. Richter, et al. (2009). "Macrophages in vaginal but not intestinal mucosa are monocyte-like and permissive to human immunodeficiency virus type 1 infection." J Virol 83(7): 3258-67.
Sherr, C. J. and J. M. Roberts (1995). "Inhibitors of mammalian G1 cyclin-dependent kinases." Genes Dev 9(10): 1149-63.
Steinman RA, J. D. (2000). "p21WAF1 prevents down-modulation of the apoptotic inhibitor protein c-IAP1 and inhibits leukemic apoptosis." Mol Med 6: 736-749.
Tanaka, H., H. Arakawa, et al. (2000). "A ribonucleotide reductase gene involved in a p53-dependent cell-cycle checkpoint for DNA damage." Nature 404(6773): 42-9.
Thelander, L. and P. Reichard (1979). "Reduction of ribonucleotides." Annu Rev Biochem 48: 133-58.
Triques, K. and M. Stevenson (2004). "Characterization of restrictions to human immunodeficiency virus type 1 infection of monocytes." J Virol 78(10): 5523-7.
Uhling U. et al (1994) " Structure of ribonucleotide reductase protein R1" Nature 370, 533-539
Ukomadu, C. and A. Dutta (2003). "p21-dependent inhibition of colon cancer cell growth by mevastatin is independent of inhibition of G1 cyclin-dependent kinases." J Biol Chem 278(44): 43586-94.
van Montfoort, N., P. A. t Hoen, et al. (2012). "Fcgamma receptor IIb strongly regulates Fcgamma receptor-facilitated T cell activation by dendritic cells." J Immunol 189(1): 92-101.
Verani, A., G. Gras, et al. (2005). "Macrophages and HIV-1: dangerous liaisons." Mol Immunol 42(2): 195-212.
Verona, R., K. Moberg, et al. (1997). "E2F activity is regulated by cell cycle-dependent changes in subcellular localization." Mol Cell Biol 17(12): 7268-82.
Xaus J, C. M., Valledor AF, Soler C, Lloberas J, Celada A (1999). "Interferon gamma induces the expression of p21waf-1 and arrests macrophage cell cycle, preventing induction of apoptosis." Immunity 11: 103-113.
Xiong Y, H. G., Zhang H, Casso D, Kobayashi R, Beach D (1993). "p21 is a universal inhibitor of cyclin kinases." Nature 366: 701-704.
Zhang, J., D. T. Scadden, et al. (2007). "Primitive hematopoietic cells resist HIV-1 infection via p21." J Clin Invest 117(2): 473-81.

## Claims

1. A RNR2 inhibitor for use in the therapeutic treatment of a human patient infected with a HIV lentivirus, wherein the RNR2 inhibitor is administered in dosage conditions that enable it to interfere with reverse transcription of the lentiviral genome in HIV infected macrophages thereby altering lentiviral replication.

2. A RNR2 inhibitor for use in the prevention of constitution of HIV reservoirs or in the therapeutic treatment of replenishment of HIV reservoirs, in differentiated non-replicating macrophages in a human patient infected with a HIV lentivirus, wherein the RNR2 inhibitor is administered in dosage conditions that enable it to impair reverse transcription of the lentiviral genome in Monocyte Derived Macrophages of the patient.

3. A RNR2 inhibitor for use in the prevention or the therapeutic treatment of dissemination of the HIV lentivirus in a human patient infected with said HIV lentivirus, in particular for the prevention or the treatment of lentivirus dissemination in peripheral organs such as lungs, brain or liver or the prevention or treatment of lesions in peripheral organs such as lungs, brain or liver, wherein the RNR2 inhibitor is administered in dosage conditions that impair reverse transcription of the lentiviral genome in Monocyte Derived Macrophages of the patient.

4. A RNR2 inhibitor for use in the prevention or therapeutic treatment of a human patient infected with a HIV lentivirus according to claim 1, wherein the targeted macrophages are differentiated non replicating macrophages, in particular Monocyte Derived Macrophages (MDMs).

5. A RNR2 inhibitor for use in the treatment of a human patient infected with a HIV lentivirus according to any of claims 1 to 4 wherein the RNR2 inhibitor is administered in dosage conditions, which do not affect Monocyte Derived Macrophages viability.

6. A RNR2 inhibitor for use in the treatment of a human patient infected with a HIV lentivirus according to any of claims 1 to 5, wherein the inhibitor is selected among radical scavengers, iron chelators, antisense oligonucleotides, and interfering RNA.

7. A RNR2 inhibitor for use in the treatment of a human patient infected with a HIV lentivirus according to any of claims 1 to 6, wherein the inhibitor is selected among Hydroxyurea, Deferoxamine mesylate, oligonucleotide GTI-2040 and small interfering RNA.

8. A RNR2 inhibitor for use in the treatment of a human patient infected with a HIV lentivirus according to any of claims 1 to 7, where the human patient has been diagnosed for early phase infection with HIV.

9. A RNR2 inhibitor for use in the treatment of a human patient infected with a HIV lentivirus according to any of claims 1 to 8, where the patient is under combination antiretroviral therapy.

10. A RNR2 inhibitor for use in the treatment of a human patient infected with a HIV lentivirus according to claim 9, in combination antiretroviral therapy (cART) with a nucleoside analog, such as dideoxyinoside (DDI) and/or with 3'-azido-3'-deoxythymidine (AZT), and/or a protease inhibitors and/or integrase inhibitors.

11. A RNR2 inhibitor for use in the treatment of a human patient infected with a HIV lentivirus according to any of claims 1 to 10, wherein the patient has been diagnosed for neurological disorders.

12. A pharmaceutical formulation comprising an inhibitor of the RNR2 subunit of the ribonucleotide reductase in an amount sufficient to inhibit HIV replication in differentiated, non-replicating macrophages.

13. A pharmaceutical formulation for use according to any of claims 1 to 11 in therapeutically treating a human patient previously diagnosed for infection by a HIV lentivirus, which formulation comprises active ingredients including a RNR2 inhibitor in combination with a pharmaceutical carrier or diluents, wherein the RNR2 inhibitor is present in the formulation in a form and amount adapted to release RNR2 inhibitor after administration to the patient, such that RNR2 inhibitor release causes RNR2 inhibitor to be detectable in the plasma of the patient in a concentration that does not induce hematological toxicity, in particular in a concentration of 0.1 to 2µM, in particular 0.2 to 1µM of the RNR2 inhibitor in plasma.

14. A pharmaceutical composition according to any of claims 12 or 13, where the RNR2 inhibitor is selected among radical scavengers, iron chelators antisense oligonucleotides and interfering RNA, in particular the RNR2 inhibitor is Hydroxyurea or Deferoxamine mesylate.

15. A method of *in vitro* screening RNR2 inhibitors candidates for their capacity to interfere with reverse transcription of a HIV or SIV lentiviral genome in human differentiated non-replicating macrophages, in particular in Monocyte Derived Macrophages, comprising the steps of:
- providing in a macrophages culture medium, differentiated Monocyte Derived Macrophages (MDMs) that express the following markers: CD14, Fcγ receptors, differentiation markers including CD11b and/or CD71 and M2 macrophage polarization markers including CD163 and/or CD206;
- infecting MDMs with a HIV retrovirus or a derivative thereof wherein said HIV or derivative optionally expresses a reporter gene;
- contacting the infected MDMs with determined concentrations of a candidate RNR2 inhibitor;
- determining HIV replication gene products, in particular measuring the expression or the activity of the reporter gene, thereby determining efficacy of RNR2 inhibitor candidate to block HIV infection of MDMs.

16. A method of *in vitro* screening RNR2 inhibitors for their capacity to interfere with reverse transcription of a HIV or SIV lentiviral genome in human differentiated non-replicating macrophages, in particular in Monocyte Derived Macrophages, according to claim 15, comprising steps for preparation of MDMs including:
- providing buffy coat of anticoagulated blood sample of a healthy donor;
- differentiating monocytes of the buffy coat into macrophages and harvesting Monocyte Derived Macrophages (MDMs) that express the following markers: CD14, Fcγ receptors, differentiation markers including C-11b and/or CD71 and M2 macrophage polarization markers including CD163 and/or CD206;
- resuspending MDMs in macrophage culture medium.

17. A method of *in vitro* screening according to any of claims 15 or 16, which further comprises a step of assaying specificity of the candidate RNR2 inhibitor for targeting RNR2.


SEQUENCE LISTING

<110>  INSTITUT PASTEUR

<120>  INHIBITORS OF RNR2 SUBUNIT OF RIBONUCLEOTIDE REDUCTASE TO IMPAIR
       REVERSE TRANSCRIPTION OF HIV RELATED LENTIVIRUSES IN MACROPHAGES
       AND USE THEREOF FOR THE TREATMENT OF HIV INFECTED PATIENTS

<130>  B10174 - AD/KN

<140>  EP XXXXXX
<141>  2013-05-21

<160>  14

<170>  PatentIn version 3.5

<210>  1
<211>  19
<212>  RNA
<213>  Artificial Sequence

<220>
<223>  Oligonucleotide - siRNR2: 1

<400>  1
gcacucuaau gaagcaaua                                                    19


<210>  2
<211>  16
<212>  RNA
<213>  Artificial Sequence

<220>
<223>  Oligonucleotide - siRNR2: 2

<400>  2
gaagaguagg gaguau                                                       16


<210>  3
<211>  19
<212>  RNA
<213>  Artificial Sequence

<220>
<223>  Oligonucleotide - siRNR2: 3

<400>  3
gaguagagaa cccauuuga                                                    19


<210>  4
<211>  19
<212>  RNA
<213>  Artificial Sequence

<220>
<223>  Oligonucleotide - siRNR2:4

<400>  4
ggcucagcuu ggucgacaa                                                    19

<210>  5
<211>  19
<212>  RNA
<213>  Artificial Sequence

<220>
<223>  Oligonucleotide - siE2F1: 1

<400>  5
gaacagggcc acugacucu                                                    19


<210>  6
<211>  19
<212>  RNA
<213>  Artificial Sequence

<220>
<223>  Oligonucleotide - siE2F1: 2

<400>  6
uggaccaccu gaugaauau                                                    19


<210>  7
<211>  19
<212>  RNA
<213>  Artificial Sequence

<220>
<223>  Oligonucleotide - siE2F1: 3

<400>  7
cccaggaggu cacuucuga                                                    19


<210>  8
<211>  19
<212>  RNA
<213>  Artificial Sequence

<220>
<223>  Oligonucleotide - siE2F1: 4

<400>  8
ggcuggaccu ggaaacuga                                                    19


<210>  9
<211>  19
<212>  RNA
<213>  Artificial Sequence

<220>
<223>  Oligonucleotide - siRB: 1

<400>  9
gaaaggacau gugaacuua                                                    19


<210>  10

<211>  19
<212>  RNA
<213>  Artificial Sequence

<220>
<223>  Oligonucleotide - siRB:2

<400>  10
gaagaaguau gauguauug                                                    19


<210>  11
<211>  19
<212>  RNA
<213>  Artificial Sequence

<220>
<223>  Oligonucleotide - siRB: 3

<400>  11
gaaaugacuu cuacucgaa                                                    19


<210>  12
<211>  19
<212>  RNA
<213>  Artificial Sequence

<220>
<223>  Oligonucleotide - siRB: 4

<400>  12
gguucaacua cgcguguaa                                                    19


<210>  13
<211>  3284
<212>  DNA
<213>  Homo sapiens

<400>  13
cccgtgcacc ctgtcccagc cgtcctgtcc tggctgctcg ctctgcttcg ctgcgcctcc      60

actatgctct ccctccgtgt cccgctcgcg cccatcacgg acccgcagca gctgcagctc     120

tcgccgctga aggggctcag cttggtcgac aaggagaaca cgccgccggc cctgagcggg     180

acccgcgtcc tggccagcaa gaccgcgagg aggatcttcc aggagcccac ggagccgaaa     240

actaaagcag ctgcccccgg cgtggaggat gagccgctgc tgagagaaaa cccccgccgc     300

tttgtcatct tccccatcga gtaccatgat atctggcaga tgtataagaa ggcagaggct     360

tccttttgga ccgccgagga ggtggacctc tccaaggaca ttcagcactg ggaatccctg     420

aaacccgagg agagatattt tatatcccat gttctggctt tctttgcagc aagcgatggc     480

atagtaaatg aaaacttggt ggagcgattt agccaagaag ttcagattac agaagcccgc     540

tgtttctatg gcttccaaat tgccatggaa aacatacatt ctgaaatgta tagtcttctt     600

attgacactt acataaaaga tcccaaagaa agggaatttc tcttcaatgc cattgaaacg     660

atgccttgtg tcaagaagaa ggcagactgg gccttgcgct ggattgggga caaagaggct 720

acctatggtg aacgtgttgt agcctttgct gcagtggaag gcattttctt ttccggttct 780

tttgcgtcga tattctggct caagaaacga ggactgatgc ctggcctcac attttctaat 840

gaacttatta gcagagatga gggtttacac tgtgattttg cttgcctgat gttcaaacac 900

ctggtacaca aaccatcgga ggagagagta agagaaataa ttatcaatgc tgttcggata 960

gaacaggagt tcctcactga ggccttgcct gtgaagctca ttgggatgaa ttgcactcta 1020

atgaagcaat acattgagtt tgtggcagac agacttatgc tggaactggg ttttagcaag 1080

gttttcagag tagagaaccc atttgacttt atggagaata tttcactgga aggaaagact 1140

aacttctttg agaagagagt aggcgagtat cagaggatgg gagtgatgtc aagtccaaca 1200

gagaattctt ttaccttgga tgctgacttc taaatgaact gaagatgtgc ccttacttgg 1260

ctgatttttt ttttccatct cataagaaaa atcagctgaa gtgttaccaa ctagccacac 1320

catgaattgt ccgtaatgtt cattaacagc atctttaaaa ctgtgtagct acctcacaac 1380

cagtcctgtc tgtttatagt gctggtagta tcaccttttg ccagaaggcc tggctggctg 1440

tgacttacca tagcagtgac aatggcagtc ttggctttaa agtgaggggt gaccctttag 1500

tgagcttagc acagcgggat taaacagtcc tttaaccagc acagccagtt aaaagatgca 1560

gcctcactgc ttcaacgcag attttaatgt ttacttaaat ataaacctgg cactttacaa 1620

acaaataaac attgtttgta ctcacaaggc gataatagct tgatttattt ggtttctaca 1680

ccaaatacat tctcctgacc actaatggga gccaattcac aattcactaa gtgactaaag 1740

taagttaaac ttgtgtagac taagcatgta atttttaagt tttattttaa tgaattaaaa 1800

tatttgttaa ccaactttaa agtcagtcct gtgtatacct agatattagt cagttggtgc 1860

cagatagaag acaggttgtg tttttatcct gtggcttgtg tagtgtcctg ggattctctg 1920

ccccctctga gtagagtgtt gtgggataaa ggaatctctc agggcaagga gcttcttaag 1980

ttaaatcact agaaatttag ggtgatctgg ggccttcata tgtgtgagaa gccgtttcat 2040

tttatttctc actgtatttt cctcaacgtc tggttgatga gaaaaaattc ttgaagagtt 2100

ttcatatgtg ggagctaagg tagtattgta aaatttcaag tcatccttaa acaaaatgat 2160

ccacctaaga tcttgcccct gttaagtggt gaaatcaact agaggtggtt cctacaagtt 2220

gttcattcta gttttgtttg gtgtaagtag gttgtgtgag ttaattcatt tatatttact 2280

atgtctgtta aatcagaaat tttttattat ctatgttctt ctagatttta cctgtagttc 2340

atacttcagt cacccagtgt cttattctgg cattgtctaa atctgagcat tgtctagggg 2400

gatcttaaac tttagtagga aaccatgagc tgttaataca gtttccattc aaatattaat 2460

ttcagaatga aacataattt tttttttttt ttttgagatg gagtctcgct ctgttgccca 2520

ggctggagtg cagtggcgcg attttggctc actgtaacct ccatctcctg ggttcaagca 2580

attctcctgt ctcagcctcc ctagtagctg ggactgcagg tatgtgctac cacacctggc 2640

taatttttgt atttttagta gagatggagt ttcaccatat tggtcaggct ggtcttgaac 2700

tcctgacctc aggtgatcca cccacctcgg cctcccaaag tgctgggatt gcaggcgtga 2760

taaacaaata ttcttaatag ggctactttg aattaatctg cctttatgtt tgggagaaga 2820

aagctgagac attgcatgaa agatgatgag agataaatgt tgatcttttg gccccatttg 2880

ttaattgtat tcagtatttg aacgtcgtcc tgtttattgt tagttttctt catcatttat 2940

tgtatagaca atttttaaat ctctgtaata tgatacattt tcctatcttt taagttattg 3000

ttacctaaag ttaatccaga ttatatggtc cttatatgtg tacaacatta aaatgaaagg 3060

ctttgtcttg cattgtgagg tacaggcgga agttggaatc aggttttagg attctgtctc 3120

tcattagctg aataatgtga ggattaactt ctgccagctc agaccatttc ctaatcagtt 3180

gaaagggaaa caagtatttc agtctcaaaa ttgaataatg cacaagtctt aagtgattaa 3240

aataaaactg ttcttatgtc agtttcaaaa aaaaaaaaaa aaaa 3284

<210> 14
<211> 389
<212> PRT
<213> Homo sapiens

<400> 14

Met Leu Ser Leu Arg Val Pro Leu Ala Pro Ile Thr Asp Pro Gln Gln
1               5                   10                  15

Leu Gln Leu Ser Pro Leu Lys Gly Leu Ser Leu Val Asp Lys Glu Asn
            20                  25                  30

Thr Pro Pro Ala Leu Ser Gly Thr Arg Val Leu Ala Ser Lys Thr Ala
        35                  40                  45

Arg Arg Ile Phe Gln Glu Pro Thr Glu Pro Lys Thr Lys Ala Ala Ala
    50                  55                  60

Pro Gly Val Glu Asp Glu Pro Leu Leu Arg Glu Asn Pro Arg Arg Phe
65                  70                  75                  80

Val Ile Phe Pro Ile Glu Tyr His Asp Ile Trp Gln Met Tyr Lys Lys
                85                  90                  95

Ala Glu Ala Ser Phe Trp Thr Ala Glu Glu Val Asp Leu Ser Lys Asp
            100                 105                 110

Ile Gln His Trp Glu Ser Leu Lys Pro Glu Glu Arg Tyr Phe Ile Ser
        115                 120                 125

His Val Leu Ala Phe Phe Ala Ala Ser Asp Gly Ile Val Asn Glu Asn
    130                 135                 140

Leu Val Glu Arg Phe Ser Gln Glu Val Gln Ile Thr Glu Ala Arg Cys
145                 150                 155                 160

Phe Tyr Gly Phe Gln Ile Ala Met Glu Asn Ile His Ser Glu Met Tyr
                165                 170                 175

Ser Leu Leu Ile Asp Thr Tyr Ile Lys Asp Pro Lys Glu Arg Glu Phe
            180                 185                 190

Leu Phe Asn Ala Ile Glu Thr Met Pro Cys Val Lys Lys Lys Ala Asp
        195                 200                 205

Trp Ala Leu Arg Trp Ile Gly Asp Lys Glu Ala Thr Tyr Gly Glu Arg
    210                 215                 220

Val Val Ala Phe Ala Ala Val Glu Gly Ile Phe Phe Ser Gly Ser Phe
225                 230                 235                 240

Ala Ser Ile Phe Trp Leu Lys Lys Arg Gly Leu Met Pro Gly Leu Thr
                245                 250                 255

Phe Ser Asn Glu Leu Ile Ser Arg Asp Glu Gly Leu His Cys Asp Phe
            260                 265                 270

Ala Cys Leu Met Phe Lys His Leu Val His Lys Pro Ser Glu Glu Arg
        275                 280                 285

Val Arg Glu Ile Ile Ile Asn Ala Val Arg Ile Glu Gln Glu Phe Leu
    290                 295                 300

Thr Glu Ala Leu Pro Val Lys Leu Ile Gly Met Asn Cys Thr Leu Met
305                 310                 315                 320

Lys Gln Tyr Ile Glu Phe Val Ala Asp Arg Leu Met Leu Glu Leu Gly
                325                 330                 335

Phe Ser Lys Val Phe Arg Val Glu Asn Pro Phe Asp Phe Met Glu Asn
            340                 345                 350

Ile Ser Leu Glu Gly Lys Thr Asn Phe Phe Glu Lys Arg Val Gly Glu
        355                 360                 365

Tyr Gln Arg Met Gly Val Met Ser Ser Pro Thr Glu Asn Ser Phe Thr

370                 375                 380

Leu Asp Ala Asp Phe
385